# EUROPEAN PATENT SPECIFICATION

(11) **EP 1 664 798 B1**
(45) Date of publication and mention of the grant of the patent: **22.05.2013**
(21) Application number: 04783513.7
(22) Date of filing: 08.09.2004
(51) Int. Cl.: G01N 33/74, A61K 31/194, A61K 31/4196, A61K 31/445, A61K 31/4453, A61K 31/45, A61P 1/00, A61P 17/00, A61P 27/02, A61P 9/08, A61P 9/10, A61P 3/10, A61P 35/00, A61P 9/02

(54) **NON-PEPTIDE AGONISTS AND ANTAGONISTS OF ADRENOMEDULLIN AND GASTRIN RELEASING PEPTIDE**
NICHT PEPTIDISCHE AGONISTEN UND ANTAGONISTEN VON ADRENOMEDULLIN UND GASTRIN FREISETZENDE PEPTIDE
AGONISTES ET ANTAGONISTES NON PEPTIDIQUES D'ADRENOMEDULLINE ET PEPTIDE DE LIBERATION GASTRIQUE

(30) Priority: 08.09.2003 US 500650 P; 11.05.2004 US 569625 P
(43) Date of publication of application: 07.06.2006
(60) Divisional application: 11000695.4
(73) Proprietor: The Government of the United States of America as represented by the Secretary of the Department of Health and Human Services, Bethesda, MD 20852 (US)
(72) Inventor: CUTTITTA, Frank, Adamstown, MD 21710 (US); MARTINEZ, Alfredo, Bethesda, MD 20814 (US)
(74) Representative: Lord, Hilton David
(86) International application number: PCT/US2004/029293
(87) International publication number: WO 2005/026741

(56) References cited:
- EP-A- 0 806 418
- WO-A-00/30630
- WO-A-97/07214
- WO-A-99/05299
- FR-A- 2 241 313
- LOOKER, J. H. ET AL: "1,2,3-Thiadiazoles as potential antineoplastic agents. I. Synthesis of novel 4-monosubstituted and 4,5-disubstituted derivatives Physiologically active compounds. III. Hydrochlorides of amino esters of phenylcyclohexylglycolic acids, of amides of benzilic, phenylcyclohexyl- and dicyclohexylglycolic," JOURNAL OF HETEROCYCLIC CHEMISTRY , 2(4), 348-54 CODEN: JHTCAD; ISSN: 0022-152X, 1965, XP002314287
- TMEJ C ET AL: "A COMBINED HANSCH/FREE-WILSON APPROACH AS PREDICTIVE TOOL IN QSAR STUDIES ON PROPAFENONE-TYPE MODULATORS OF MULTIDRUG RESISTANCE" ARCHIV DER PHARMAZIE, VCH VERLAGSGESELLSCHAFT MBH, WEINHEIM, DE, vol. 331, no. 7/8, 1998, pages 233-240, XP000881287 ISSN: 0365-6233
- MURAD S ET AL: "STRUCTURE-ACTIVITY RELATIONSHIP OF MINOXIDIL ANALOGS AS INHIBITORS OF LYSYL HYDROXYLASE IN CULTURED FIBROBLASTS1" ARCHIVES OF BIOCHEMISTRY AND BIOPHYSICS, NEW YORK, US, US, vol. 292, no. 1, 1992, pages 234-238, XP000564071 ISSN: 0003-9861
- EAKIN A E ET AL: "COMPARATIVE COMPLEMENT SELECTION IN BACTERIA ENABLES SCREENING FOR LEAD COMPOUNDS TARGETED TO A PURINE SALVAGE ENZYME OF PARASITES" ANTIMICROBIAL AGENTS AND CHEMOTHERAPY, AMERICAN SOCIETY FOR MICROBIOLOGY, WASHINGTON, DC, US, vol. 39, no. 3, March 1995 (1995-03), pages 620-625, XP001055590 ISSN: 0066-4804
- MARTÍNEZ A ET AL: "Identification of vasoactive nonpeptidic positive and negative modulators of adrenomedullin using a neutralizing antibody-based screening strategy" ENDOCRINOLOGY 2004 UNITED STATES, vol. 145, no. 8, August 2004 (2004-08), pages 3858-3865, XP009042699 ISSN: 0013-7227
- GASPARETTO M ET AL: "Identification of compounds that enhance the anti-lymphoma activity of rituximab using flow cytometric high-content screening" JOURNAL OF IMMUNOLOGICAL METHODS, ELSEVIER SCIENCE PUBLISHERS B.V.,AMSTERDAM, NL, vol. 292, no. 1-2, September 2004 (2004-09), pages 59-71, XP004550500 ISSN: 0022-1759
- EGUCHI SATORU ET AL: "Structure-activity relationship of adrenomedullin, a novel vasodilatory peptide, in cultured rat vascular smooth muscle cells" ENDOCRINOLOGY, BALTIMORE, MD, US, vol. 135, no. 6, 1994, pages 2454-2458, XP002193142 ISSN: 0013-7227
- AMIR S M ET AL: "POLYPHLORETIN PHOSPHATE AN ANTAGONIST OF THYROTROPIN ACTION EVIDENCE FOR AN INTERACTION WITH THE HORMONE RATHER THAN THE RECEPTOR" ENDOCRINOLOGY, vol. 117, no. 3, 1985, pages 860-868, XP009042800 ISSN: 0013-7227
- HEUSER MARKUS ET AL: "The inhibition of angiogenesis and microcirculation in renal cell carcinoma spheroids by gastrin releasing peptide receptor (GRP-R) antagonism depends on the time of treatment initiation." JOURNAL OF UROLOGY, vol. 169, no. 4 Supplement, April 2003 (2003-04), page 207, XP009042804 & 98TH ANNUAL MEETING OF THE AMERICAN UROLOGICAL ASSOCIATION (AUA); CHICAGO, IL, USA; APRIL 26-MAY 02, 2003 ISSN: 0022-5347
- DATABASE CA [Online] CHEMICAL ABSTRACTS SERVICE, COLUMBUS, OHIO, US; 1964, CHERNOV, V. A. ET AL: "Antiblastomic activity of sydnone imines" XP002331366 retrieved from STN Database accession no. 1964:406942 & DOKLADY AKADEMII NAUK SSSR , 155(1), 216-19 CODEN: DANKAS; ISSN: 0002-3264, 1964,
- DAENIKER, H. U. ET AL: "Chemotherapeutic studies in the heterocyclic series. XVIII. Polymethylenedi(sydnones) and polymethylenedi(hydrazines)" HELVETICA CHIMICA ACTA , 40, 918-32 CODEN: HCACAV; ISSN: 0018-019X, 1957, XP009048688
- THOMAS F ET AL: "ANTITUMORAL ACTIVITY OF BOMBESIN ANALOGUE ON SMALL CELL LUNG CANCER XENOGRAFTS RELATIONSHIP WITH BOMBESIN RECEPTOR EXPRESSION" CANCER RESEARCH, vol. 52, no. 18, 1992, pages 4872-4877, XP001206441 ISSN: 0008-5472
- BAJO A M ET AL: "BOMBESIN ANTAGONISTS INHIBIT PROANGIOGENIC FACTORS IN HUMAN EXPERIMENTAL BREAST CANCERS" BRITISH JOURNAL OF CANCER, LONDON, GB, vol. 90, no. 1, 12 January 2004 (2004-01-12), pages 245-252, XP001183666 ISSN: 0007-0920
- CUTTITTA F ET AL: "BOMBESIN-LIKE PEPTIDES CAN FUNCTION AS AUTOCRINE GROWTH FACTORS IN HUMAN SMALL-CELL LUNG CANCER" NATURE (LONDON), vol. 316, no. 6031, 1985, pages 823-826, XP001206814 ISSN: 0028-0836
- ASHWOOD V ET AL: "PD 176252 - The First High Affinity Non-peptide Gastrin-Releasing Peptide (BB2) Receptor Antagonist" BIOORGANIC & MEDICINAL CHEMISTRY LETTERS, OXFORD, GB, vol. 8, no. 18, 22 September 1998 (1998-09-22), pages 2589-2594, XP004138276 ISSN: 0960-894X

## Description

### FIELD OF THE INVENTION

This invention relates generally to small molecule, non-peptide, modulators *(e.g.,* antagonists or agonists) of peptide hormones. Also described are complexes comprising such small molecules, methods of identifying the molecules as modulatory agents, and methods of diagnosis or treatment, using the molecules.

### BACKGROUND INFORMATION

Adrenomedullin (AM) is a peptide hormone implicated in the pathophysiology of important diseases such as hypertension, cancer, and diabetes. AM is a 52 amino acid peptide that belongs to the calcitonin/calcitonin gene related peptide (CGRP)/amylin/AM superfamily. In humans, this peptide is expressed by many cell types and exerts a variety of physiological roles, including vasodilatation, bronchodilatation, regulation of hormone secretion, neurotransmission, antimicrobial activities, regulation of growth, apoptosis, migration, and angiogenesis, among others.

These activities are mediated by a complex receptor system encompassing a seven transmembrane domain polypeptide known as calcitonin receptor-like receptor (CRLR), a single transmembrane domain protein, termed receptor activity modifying protein (RAMP), and the intracellular receptor component protein (RCP). RCP is necessary for the initiation of the signal transduction pathway. Three RAMPs have been identified in mammals and their coexpression with CRLR results in different binding affinities, with RAMP1 producing a characteristic CGRP-1 response whereas coexpression of CRLR with RAMP2 or RAMP3 elicits a specific AM receptor.

Gastrin releasing hormone (GRP) is a peptide hormone implicated in the pathophysiology of important diseases such as cancer and respiratory problems in premature babies. GRP is a 27 amino acid peptide, initially identified as the human counterpart of bombesin, a peptide found in the frog's skin. GRP has a variety of physiological roles. For example, it has antimicrobial properties, reduces food intake, and has been involved in respiratory development, and in the regulation of short-term memory, among others.

Several types of antagonists have been proposed for peptide hormones, including monoclonal antibodies and inhibitory peptide fragments, such as AM(22-52), AM(16-31), AM(11-26), and proAM(153-185). While these molecules are effective as research tools, they sometimes exhibit significant limitations as pharmaceutical agents, *e.g.,* because of the lack of humanized blocking antibodies and the short biological half-life of fragmentary peptides. There is a need for additional agents that modulate activities of peptide hormones, in particular AM and GRP, and that can be used to treat disease conditions mediated by the peptide hormones, such as the conditions noted above. Small molecule, non-peptide agents would be particularly desirable.

Relevant prior art includes Looker JH et al., Journal of Heterocyclic Chemistry, 1965, 2(4): 348-354; Tmej C et al., Archiv Der Pharmazie, 1998, 331(7/8): 233-240; Murad S et al., Archives of Biochemistry and Biophysics, 1992, 292(1): 234-238; Eakin AE et al., Antimicrobial Agents and Chemotherapy, 1995, 39(3): 620-625; FR 2241313; Martinez A et al., Endocrinology (2004), 145(8): 3858-3865; and Nakajima & Anselme, J. Org. Chem. (1983) Vol. 48, p.1444-1448.

According to the present invention there are provided complexes, pharmaceutical compositions, compounds for use and methods as defined in the appended independent claims. Further preferable features are defined in the appended dependent claims.

### BRIEF DESCRIPTION OF THE DRAWINGS

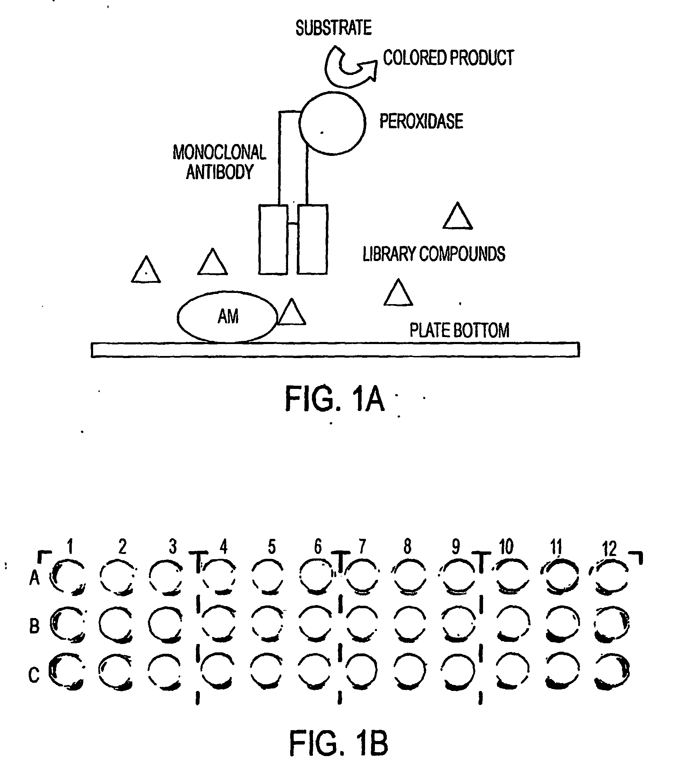

**Figure** 1 illustrates primary screen (step #1 of the method) using a blocking monoclonal antibody. **Figure 1A** shows a schematic representation of the primary screening process. **Figure 1B** shows a photograph of part of a fully developed AM-coated 96-well plate used for the initial screening of the library. Wells A1 and A2 are not coated with AM and provide the value for non-specific background. Wells A3 and A4 have been exposed to all the reagents but the competitors and their color value provides the maximum binding for the assay. Wells A5 and A6 have been exposed to 1.2 µg/ml unlabeled monoclonal antibody and constitute a positive-competition control. Individual small molecules from the library were assayed in duplicates in wells B and C. Wells B 10 and C10 contain compounds VIII (697165), one of the successful competitors. Wells A7-A12 are empty. Actual absorbance values were quantified in a plate reader.

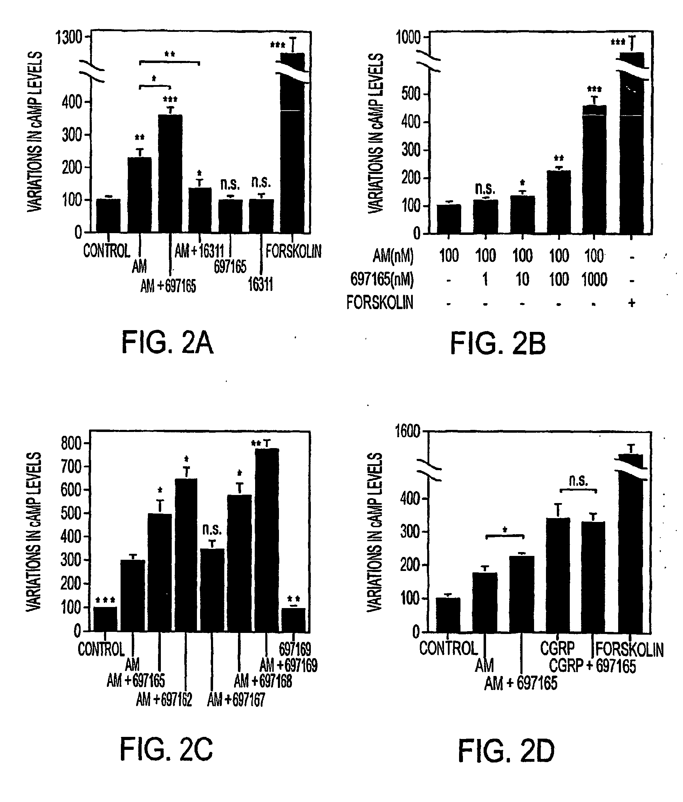

**Figure 2** shows a secondary screen (step #2 of the method) for AM-active compounds. This figure shows secondary screening of promising compounds by induction of intracellular cAMP levels in Rat2 cells **(****Figures 2A- 2C****)** and in HEK 293 cells transfected with CRLR and RAMP1 **(****Figure 2D****),** cAMP levels were quantified by radioimmunoassay and are represented as variations from the value of the first bar, arbitrarily expressed as 100. **Figure 2A** shows variations on intracellular cAMP levels induced by a superagonist compound (compound VIII, or 697165) and an antagonist (compound VI, or 79422) in the presence and absence of 100 nM AM. Forskolin was added as a positive control. Asterisks represent statistical significance when compared to the untreated control (first bar) or as indicated by the horizontal bars. **Figure 2B** shows dose-dependent elevation of cAMP induced by the superagonist compound VIII in the presence of 100 nM AM. Asterisks represent statistical significance when compared to addition of AM alone (first bar). **Figure 2C** shows a comparison of the effects elicited by other members of the family of compound VIII in the presence of 100 nM AM. Asterisks represent statistical significance when compared to addition of AM alone (second bar). **Figure 2D** shows the lack of effect of several compounds in the presence of 100 nM CGRP in the activation of the CGRP receptor in HEK 293 cells. Asterisks represent statistical significance when compared to addition of CGRP alone (second bar). Bars represent mean ± standard deviation of three independent determinations. n.s.: No significant differences; * :*p*<0.05; ** :*p*<0.01; ***: p<0.001.

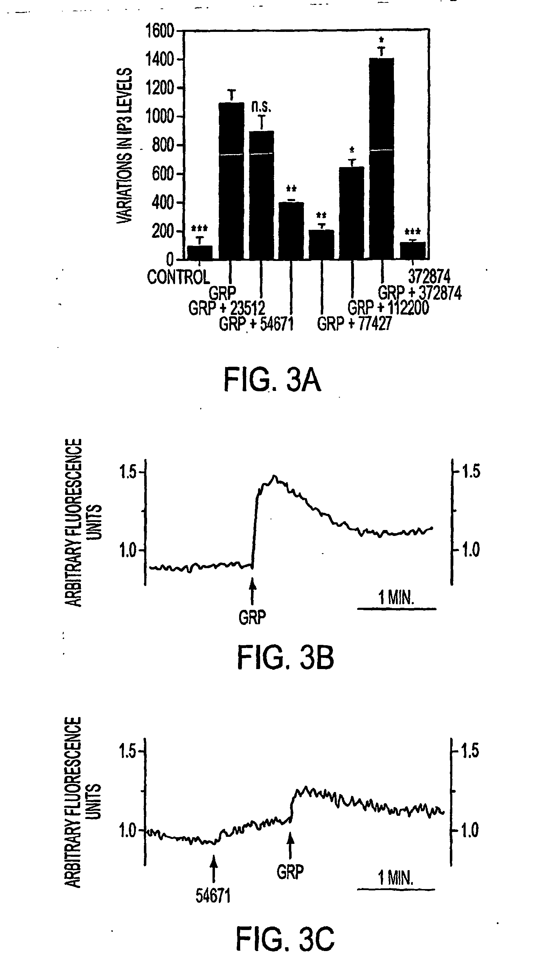

**Figure 3** shows a secondary screen for GRP-active compounds. This figure shows an analysis of second messengers for compounds that interfere with GRP binding. **Figure 3A** shows a quantification of IP3 levels in cell line H1299 exposed to different compounds in the presence or absence of 100 nM GRP. Bars represent mean ± standard deviation of three independent determinations. Asterisks represent statistical significance when compared to addition of GRP alone (second bar). n.s.: No significant differences; * : p<0.05; ** : p<0.01; ***: *p*<0.001. **Figure 3B** shows Ca²⁺ response induced by 1 nM GRP in H1299 cells. **Figure 3C** shows that preincubation of H1299 cells with compound XIV (54671) for 1 min. dramatically reduces the Ca²⁺ response elicited by 1 nM GRP.

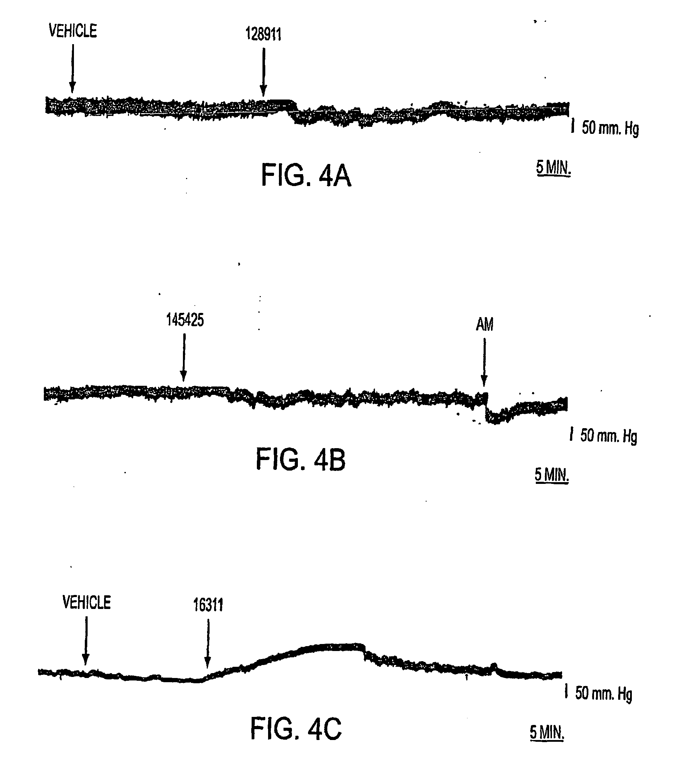

**Figure 4** shows blood pressure regulation by AM-active compounds. This figure shows representative blood pressure recordings in hypertensive SHR (A,B) and in normotensive Lewis/ssncr (C) rats following the intravenous injection of AM antagonists (XII', or 128911; XIII', or 145425), agonists (I', or 16311), or vehicle (PBS+DMSO). Synthetic AM was added in B for comparison purposes.

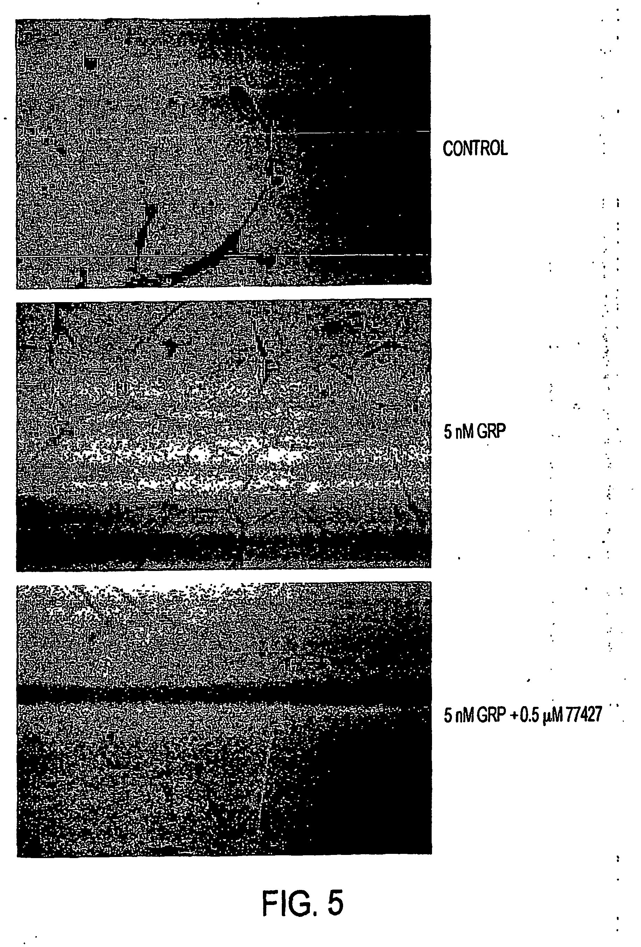

**Figure 5** shows the antiangiogenic effect of a GRP antagonist. This figure shows cord formation assay in matrigel with bovine retinal microvascular endothelial cells. Top panel shows a negative control with no additions. Middle panel shows that a complex tubular lattice is induced by 5 nM GRP. Bottom panel shows that the simultaneous addition of antagonist compound XV' (77427) (0.5 µM) reduces network complexity.

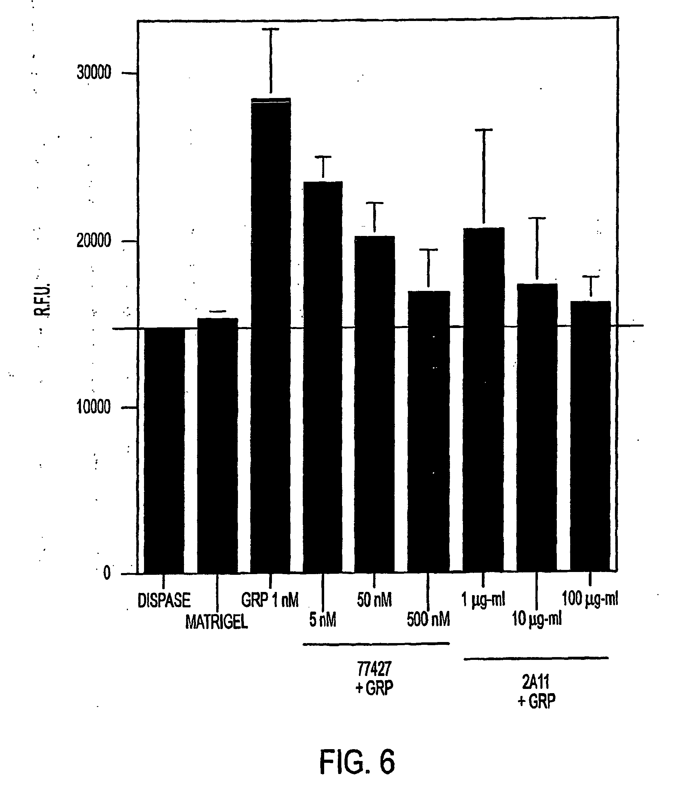

**Figure 6** shows a directed *in vivo* angiogenesis assay (DIVAA). Bars represent mean ± standard deviation of five independent determinations.

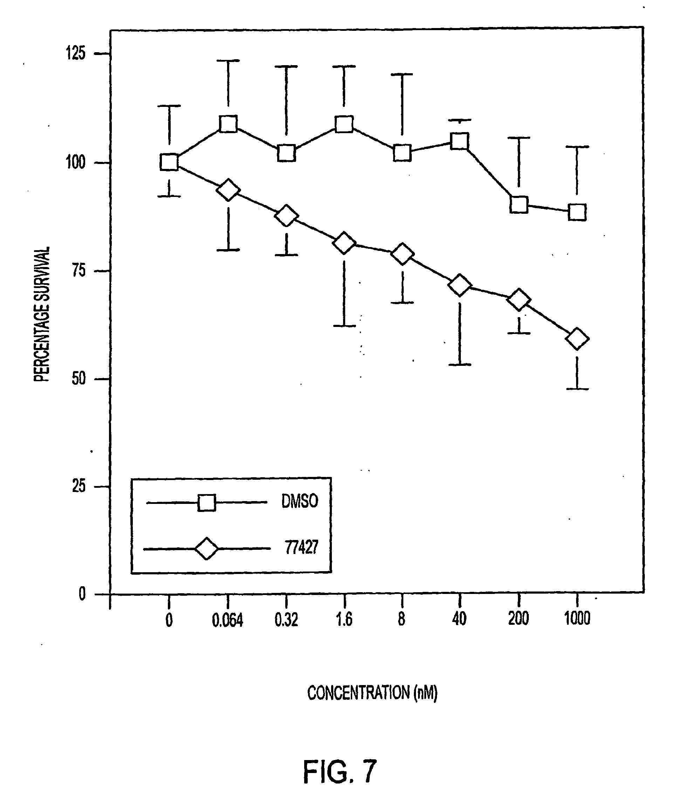

**Figure 7** shows a growth inhibition assay (MTT). Bars represent mean ± standard deviation of eight independent determinations.

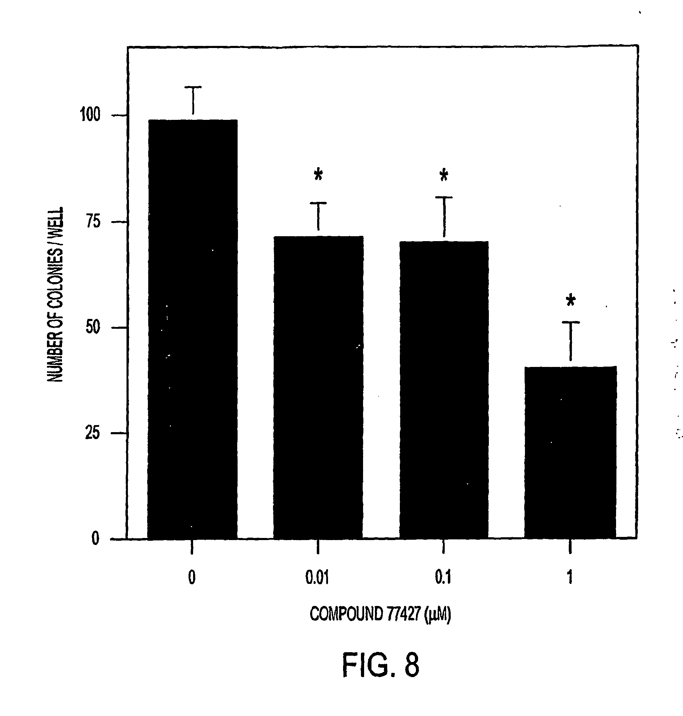

**Figure 8** shows another growth inhibition assay (clonogenic). Bars represent mean ± standard deviation of three independent determinations. * : *p*<0.05.

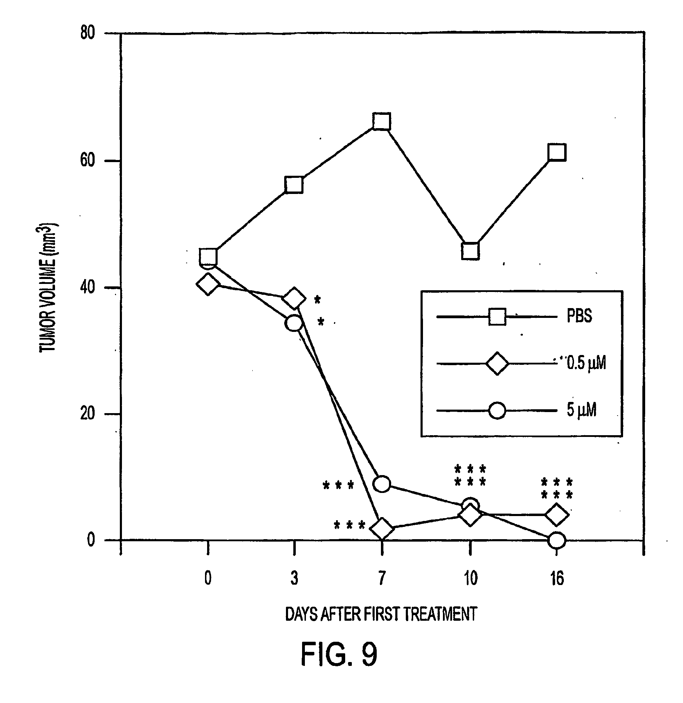

**Figure 9** shows a xenograft model in nude mice injected with cell line H1299, and treated with a small molecule inhibitor of the invention. Each point represents the mean of 10 animals. *** : *p<0.05;* ***:p<0.001.

### DESCRIPTION OF THE INVENTION

This invention relates, *e.g.,* to agents, particularly small molecule, non-peptide, agents, that modulate activities of peptides which interact with specific receptors. In preferred embodiments, the peptides whose activities are modulated are peptide hormones, most preferably adrenomedullin (AM) or gastrin releasing peptide (GRP).

As used herein, and unless otherwise specified, "aryl" includes substituted and unsubstituted monocyclic and polycyclic ring systems containing one or more aromatic rings. Aryl includes carbocyclic ring systems and a heterocyclic ring systems containing one or more heteroatoms selected from N, S and O. Exemplary carbocyclic ring systems include benzene (phenyl) and naphthalene. Examples of heteroaromatic rings include, for example, pyridine, pyrrole, furan, thiophene, indole, isoindole, benzofuran, benzothiophene, quinoline, isoquinoline, imidazole, oxazole, thiazole, purine, pyrimidine, etc. Aryl groups further include polycyclic ring systems wherein at least one of the rings is aromatic, such as, for example, indan and 1,2,3,4-tetrahyydonapthalene. In aryl groups having non-aromatic rings, the preferred point of attachment is to the aromatic ring. Preferred aryl groups contain one or two aromatic rings.

As used herein, and unless otherwise specified, the term "hydrocarbyl" includes alkyl, alkenyl and alkynyl groups, including straight chained, branched, cyclic and combinations thereof. Alkyl groups include, for example, straight chain groups such as methyl, ethyl, n-propyl, n-butyl, n-pentyl, n-hexyl, etc.; branched chains such as iso-propyl, iso-butyl, sec-butyl, tert-butyl, iso-pentyl, sec-pentyl, neopentyl, etc.; carbocyclic groups such as cylopropyl, cyclobutyl, cyclopentyl; and substituted carbocyclic groups such as methyl-, ethyl-, propyl-, etc. substituted carbocycles. Unless indicated otherwise, propyl, butyl, etc. include both straight chain and branched combinations; for example, propyl includes both n-propyl and isopropyl. Alkenyl and alkynyl groups as used herein are alkyl groups that include one or more double or triple bonds, respectively. As used herein, "lower alkyl" refers to straight chain and branched alkyl groups with four or fewer carbons; and "lower alkenyl" and "lower alkynyl" refer to double and triple bond, respectively, containing straight chain and branched groups with two to four carbon atoms. As used herein, "aromatic rings" comprise 3-7, preferably 5-6 membered rings.

As used herein, and unless otherwise specified, the term "heterocyclic ring" refers to mono- and poly-cyclic ring systems wherein a heteroatom such as N, O or S is included in at least one position of at least one ring. Heterocyclic rings may include one or more double bonds. Heterocyclic rings include, for example, pyrroline, pryrrolidine, tetrahydrofuran, tetrahydrothiophene, piperidine, morpholine, piperazine, piperolidine, etc.

As used herein, and unless otherwise specified, the term "halogen" includes the group seven atoms fluorine, chlorine, bromine, and iodine.

The present inventors have developed a two-step screening method to identify such modulatory agents. AM and GRP were used as exemplary peptide hormones in the screening assay; a variety of other peptide hormones can, of course, also be used. The term "modulate," as used herein, includes to increase, stimulate, augment, enhance, facilitate, or potentiate, or to decrease, inhibit, suppress, interfere with, prevent, block, etc. An agent that augments the activity of a peptide hormone is said to be an agonist (in some cases, as discussed below, a superagonist); an agent that suppresses the activity is said to be an antagonist. Both AM and GRP exhibit a variety of "activities," some of which are described elsewhere herein.

To identify modulatory compounds, a library of known small molecule, non-peptide, compounds was screened. Compounds were first identified on the basis of their ability to interfere with binding between AM or GRP and their respective blocking antibodies. Compounds identified as "positive" in this first step were further screened for their ability to influence receptor-mediated biological activities (inhibition of the induction of second messengers). Using this two-step procedure, seven compounds were identified as antagonists of AM, and three as antagonists of GRP. Surprisingly, in view of the fact that the compounds were first identified because of their ability to *inhibit* the binding of the peptide to its blocking antibody, other compounds were identified that act as agonists *(e.g.,* superagonists) of the peptides. Six superagonists were identified for AM, and one for GRP. A total of 17 modulatory agents were identified.

Among the advantages of the identified small molecule, non-peptide, modulatory agents are that the molecules are stable, especially when in an organism; are small and thus exhibit good cell permeability characteristics; and are readily synthesized, allowing for the rapid, inexpensive production of large quantities.

In one embodiment, the invention relates to a method for modulating an activity of an adrenomedullin (AM) peptide, comprising contacting the peptide with an effective amount of a compound of at least one of formulas I-VII, XII or XIII or a pharmaceutically acceptable salt thereof, where formulas I-VII, XII and XIII are as described below: 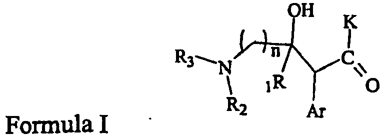 wherein:
K is OR₃₂ or SR₃₂, where R₃₂ is H or lower alkyl or K is NR₃₃R34, where R₃₃ and R₃₄ are the same or different and each is selected from H and lower alkyl. In particular embodiments, K is OH, SH or NH₂;
Ar is aryl, optionally substituted aryl optionally substituted with one or more groups selected from -OH, -NH₂, -SH, halogen and hydrocarbyl. Ar is unsubstituted in some embodiments and in other embodiments is a benzene ring optionally substituted with one or more hydrocarbyl groups and/or halogens;
n is an integer from 1-3; and
R₁, R₂ and R₃ are the same or different and is each selected from H, hydrocarbyl and a heterocyclic ring. Alternatively, R₁ and R₂ together form a heterocyclic ring including the intervening nitrogen, for example R₁ and R₂ can be -(CH₂)_{z}-, where z is an integer from 1-3. In other embodiments, R₁ is selected from H or hydrocarbyl and R₂ and R₃ together form a heterocyclic ring including the intervening nitrogen. In some embodiments, n, R₁ and R₂ are selected to form a five or six membered nitrogen containing ring.

In some embodiments, hydrocarbyl is lower alkyl. 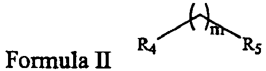 wherein:
R₄ and R₅ may be the same or different and each is an aryl group substituted with -C(O)K, and optionally further substituted with one or more groups selected from -OH, -NH₂, -SH, halogen, hydrocarbyl and a heterocyclic ring, where K is as defined above. R₄ and/or R₅ are each substituted only with -C(O)K in different positions in some embodiments, and in other embodiments each a benzene ring further substituted with one or more hydrocarbyl groups and/or halogens; and
m is an integer from 1-3.

In particular embodiments, the C(O)K group is attached to the 2- or 4-position of the aryl group, relative to the rest of the compound; and in embodiments the -C(O)K group is attached to R₄ at a different position than the -C(O)K group attached to R₅. In some embodiments, hydrocarbyl is lower alkyl. 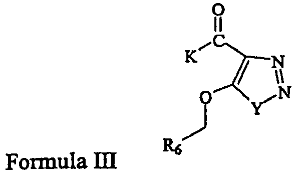 wherein:
K is as defined above;
Y is selected from CH₂, O, S and NH; and
R₆ is aryl optionally substituted with one or more groups selected from -OH, -NH₂, -SH, halogen, hydrocarbyl and a heterocyclic ring. R₆ is unsubstituted in some embodiments and in other embodiments is a benzene ring optionally substituted with one or more hydrocarbyl groups and/or halogens. 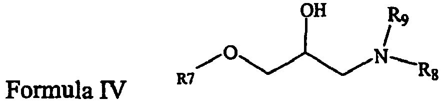 wherein:
R₇ is aryl optionally substituted with one or more groups selected from -OH, -NH₂, -SH, halogen, a heterocyclic ring and hydrocarbyl. R₇ is unsubstituted in some embodiments and in other embodiments is a benzene ring optionally substituted with one or more hydrocarbyl groups and/or halogens; and
R₈ and R₉ are the same or different and are each hydrocarbyl, optionally substituted with one or more halogens or lower alkyl groups or R₈ and R₉ together form a heterocyclic ring having five, six or seven atoms, including the intervening nitrogen and optionally containing other heteroatoms, and also optionally substituted with one or more halogens or lower alkyl groups. In particular embodiments, R₈ and R₉ together are -(CH₂)ₙ-, wherein n is 4 or 5, thus forming form a five or six membered ring including the intervening nitrogen. 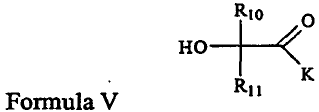 wherein:
K is as defined above; and
R₁₀ and R₁₁ are the same or different and each is an aryl group, optionally substituted with a second aryl group that may be the same or different. In some embodiments, one or both of R₁₀ and R₁₁ is a benzene ring substituted in the 2- or 4-position with an aryl group. The first or second aryl groups may be substituted with one or more groups selected from -OH, -NH₂, -SH, halogen, a heterocyclic ring and hydrocarbyl. 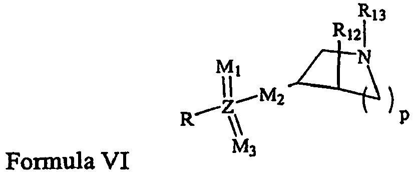 wherein:
p is an integer from 1-3;
M₁, M₂, and M₃ are the same or different and each is S or O;
Z is S, C or P;
R is hydrocarbyl or OR₃₅, where R₃₅ is H or hydrocarbyl. In some embodiments, R or R₃₅ are lower alkyl; and
R₁₂ and R₁₃ are the same or different and each is hydrocarbyl, a heterocyclic ring or lower alkyl, or R₁₂ and R₁₃ together form a ring. In particular embodiments, R₁₂ and R₁₃ together form the group -(CH₂)_{b}-, wherein b is and integer from 1-3. 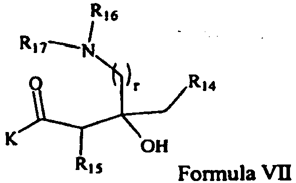
wherein:
K is as defined above;
r is an integer from 1-3;
R₁₄ and R₁₅ are the same or different and each is aryl optionally substituted with one or more groups selected from -OH, -NH₂, -SH, halogen, a heterocyclic ring and hydrocarbyl. R₁₄ and/or R₁₅ are unsubstituted in some embodiments and in other embodiments each is a benzene ring optionally substituted with one or more hydrocarbyl groups and/or halogens; and
R₁₆ and R₁₇ are the same or different and each is hydrocarbyl or a heterocyclic ring. In particular embodiments, R₁₆ and R₁₇ are the same or different and each is lower alkyl. 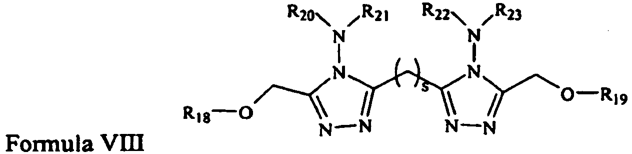
wherein:
s is an integer from 1-10 and, in particular embodiments is an integer from 6-8;
R₂₀, R₂₁, R₂₂ and R₂₃ are the same or different and each is H, aryl, optionally substituted with one or more of halogen, lower alkyl, hydrocarbyl or a heterocyclic ring; and
R₁₈, R₁₉ are the same or different and each is aryl optionally substituted with one or more groups selected from -OH, -NH₂, -SH, halogen or lower alkyl. 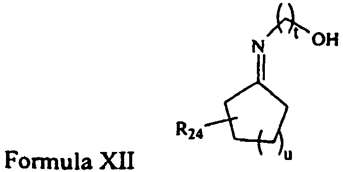
wherein:
t is an integer from 1-5;
u is an integer from 1-2; and
R₂₄ is selected from H, a heteroyclic ring and hydrocarbyl, optionally substituted by halogen. In particular embodiments, R₂₄ is H or lower alkyl. 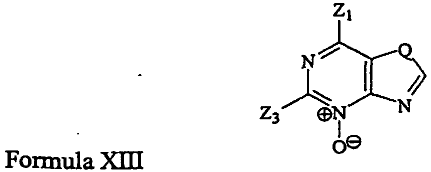
wherein:
Q is selected from CH₂, NH, S and O; and
Z₁ and Z₃ are the same or different and selected from CH₃, NH₂, OH and SH. Formula XIII also includes tautomers of the illustrated structure.

More particularly, a compound of a formula as below may be used: 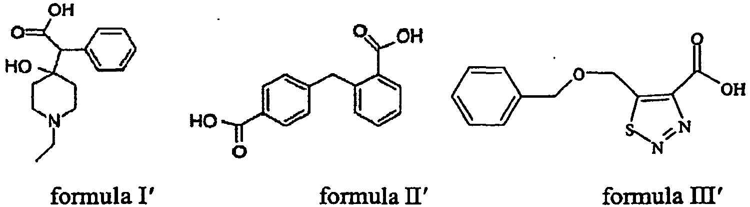 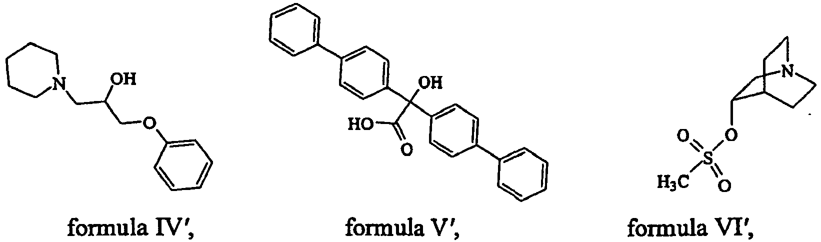 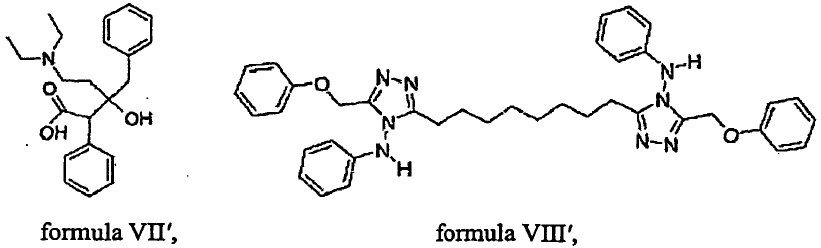 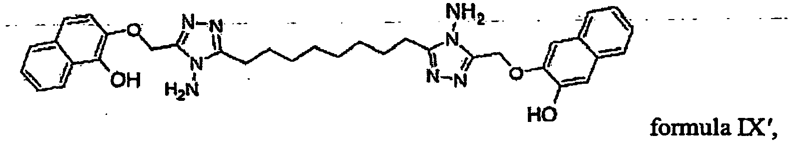 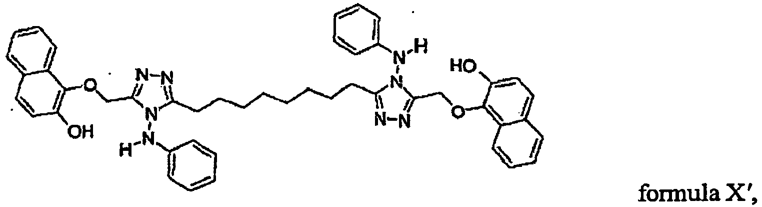 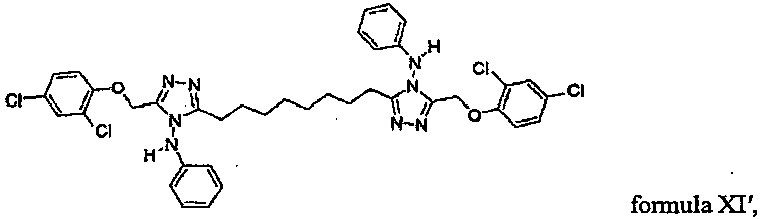 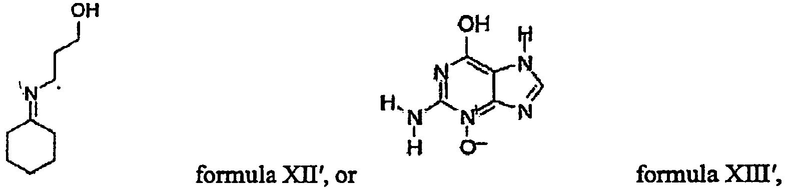 or a pharmaceutically acceptable salt thereof.

The discussion herein sometimes refers to a compound as having a structure of formula I-VIII, XII or XIII, or formula r - XIII'. It is to be understood that, unless the context clearly dictates otherwise, a pharmaceutically acceptable salt of the compound is also included.

In one embodiment, the modulation is the *inhibition* of an AM peptide activity, and the compound is represented by one of formula I through formula VII or, more particularly, the compound is represented by one of formula 1'through formula VII'. The activity that is inhibited may be, *e.g*., stimulation of the level of intracellular cAMP, vasodilation, or the like. Another embodiment is a method for treating a condition that is mediated by over-expression and/or -activity of AM, comprising administering to a patient in need of such treatment an effective amount of a compound of formula I, **II,** III, IV, V, VI, VII, I', II', III', IV', V', VI', or VII'. Among suitable conditions for such treatment are type 2 diabetes or cancer.

In another embodiment, the modulation is the *stimulation* of an AM peptide activity, and the compound is represented by one of formula VIII, XII or XIII or, more particularly, the compound is represented by one of compound VIII', IX', X', XI', XII' or XIII'. The activity that is inhibited may be, *e.g*., stimulation of the level of intracellular cAMP, vasodilation, or the like. Another embodiment is a method for treating a condition that is mediated by under-expression and/or -activity of AM, comprising administering to a patient in need of such treatment an effective amount of a compound of formula VIII, XII, XIII, VIII', IX', X', XI', XII' or XIII'. Among suitable conditions for such treatment are renal or cardiovascular disease, sepsis, or central nervous system ischemia.

In embodiments of the preceding methods to inhibit or stimulate AM, the peptide and the compound are in an animal, such as a mammal *(e.g.,* following the administration of the compound to the animal *in vivo),* or the peptide and the compound are *in vitro* (not in an animal).

In another embodiment, the invention relates to a method for modulating an activity of a gastrin releasing peptide (GRP) peptide, comprising contacting the peptide with an effective amount of a compound of at least one of Formulas XIV-XVII, or a pharmaceutically acceptable salt thereof, where formulas XIV-XVII are: 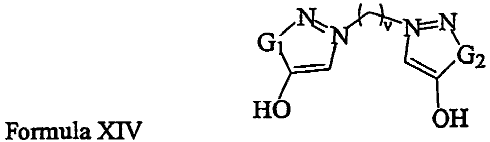 wherein:
v is an integer from 1-3; and
G₁ and G₃ are the same or different and each is selected from CH2, NH, S and O. 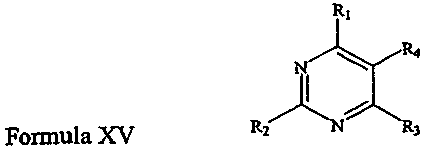
wherein
R₁ is: -R₅-(CH₂)ₙ-CH(R₆)OH, and R₅ is NH, S or O, R₆ is H or CH₃; and n is an integer from 1-4;
R₂ is NH₂, substituted amino or acetamide;
R₃ is H, halogen, CH₃, or CF₃; and
R₄ is H, alkyl, substituted alkyl, alkenyl, alkoxy or halogen, 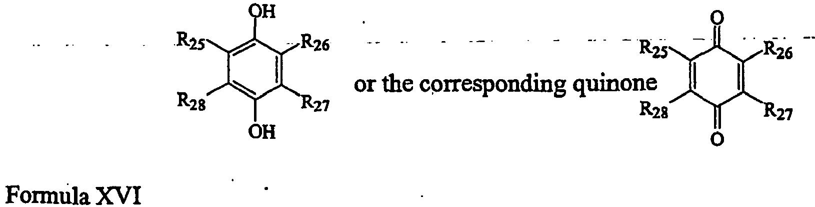
wherein:
R₂₅, R₂₆, R₂₇, and R₂₈ are the same or different and each is selected from halogen and hydrocarbyl, particularly lower alkyl. In particular embodiments, R₂₅= R₂₇, and R₂₆= R₂₈. In further embodiments, R₂₅ and R₂₇ are each halogen and R₂₆ and R₂₈ are each lower alkyl; 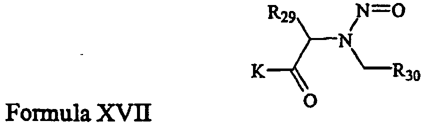
wherein:
K is as defined above, and
R₂₉ and R₃₀ are the same or different and each is aryl optionally substituted with one or more groups selected from -OH, NH₂, -SH, halogen and hydrocarbyl. R₂₉ and/or R₃₀ are unsubstituted in some embodiments and in other embodiments a benzene ring optionally substituted with one or more a hydrocarbyl groups and/or halogens.

More particularly, a compound of a formula as below is used: 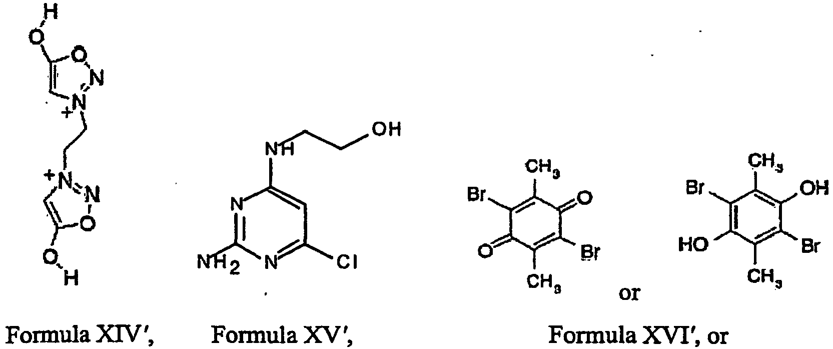 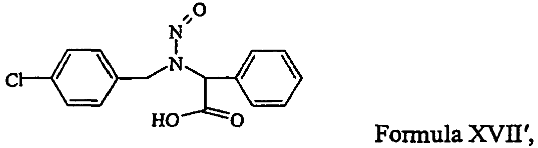 or a pharmaceutically acceptable salt thereof.

The discussion herein sometimes refers to a compound as having a structure of formula XIV-XVII, or formula XIV'- XVII'. It is to be understood that, unless the context clearly dictates otherwise, a pharmaceutically acceptable salt of the compound is also included.

In one embodiment, the modulation is the *inhibition* of a GRP peptide activity, and the compound is represented by one of formula XIV - XVI or, more particularly, the compound is represented by one of formula XIV' - XVI'. The activity that is inhibited may be, *e.g*., suppressing food intake, regulating glucose homeostasis, or stimulating hypotension. In another embodiment, the compound is represented by formula XIV, XIV', XVI or XVT, and the activity that is inhibited is, *e.g*., stimulating intracellular levels of IP₃ or Ca⁺², or stimulating angiogenesis. Another embodiment is a method for treating a condition that is mediated by over-expression and/or -activity of GRP, comprising administering to a patient in need of such treatment an effective amount of a compound of formula XIV, XV, XVI, XIV', XV', or XVI'. Among suitable treatment methods are treating low blood pressure (hypotension) or an eating disorder (such as anorexia or bulimia), or improving breathing in premature babies (bronchopulmonary dysplasia). In another embodiment, the compound is of formula XIV, XIV', XVI or XVI', and the treatment method is, *e.g.,* reducing tumor growth.

In another embodiment, the modulation is the *stimulation* of a GRP peptide activity, and the compound is represented by formula XVII or, more particularly, by formula XVII'. The activity that is inhibited may be, *e.g.,* stimulating intracellular levels of IP3 (inositol phosphate) or Ca⁺², or stimulating angiogenesis, suppressing food intake, regulating glucose homeostasis, or stimulating hypotension. Another embodiment is a method for treating a condition that is mediated by under-expression and/or -activity of GRP, and/or that would benefit from an increased expression or activity of a GRP activity (such as angiogenesis), comprising administering to a patient in need of such treatment an effective amount of a compound of formula XVII or XVII'. Among suitable conditions for such treatment are obesity, diabetes or hypertension. Furthermore, the method may be a method for treating a condition in which stimulation of angiogenesis is desirable, *e.g*., coronary or peripheral artery disease, tissue ischemia, organ or tissue transplantation, and acceleration or enhancing of fracture repair or wound healing.

In embodiments of the preceding methods to inhibit or stimulate GRP, the peptide and the compound are in an animal, such as a mammal *(e.g.,* following the administration of the compound to the animal *in vivo*)*,* or the peptide and the compound are *in vitro* (not in an animal).

In another embodiment, the invention relates to a complex, comprising a compound selected from formula I through VIII, XII, or XIII (or, more particularly; formula I' through formula XIII'), in association with (*e.g*., bound to) an AM peptide, or comprising a compound selected from formula XIV through formula XVII (or, more particularly, formula XIV' through XVII'), in association with *(e.g.,* bound to) a GRP peptide. The complex may be in an animal, such as a mammal *(e.g.,* following the administration of the compound to the animal *in vivo*)*,* or it may be *in vitro* (not in an animal).

In another embodiment, the invention relates to a complex comprising a compound selected from formula I through VIII, XII, or XIII (or, more particularly, formula I'through formula XIII'), in association with (e.g., bound to) a blocking antibody of AM, or comprising a compound selected from formula XIV to formula XVII (or, more particularly, formula XIV' through XVII'), in association with *(e.g.,* bound to) a blocking antibody of GRP. The complex may be in an animal, such as a mammal, or it may be *in vitro* (not in an animal).

In another embodiment, the invention relates to a composition, comprising a compound selected from formula I through VIII, XII, or XIII (or, more particularly, formula I'through formula XIII'), in association with *(e.g.,* bound to) an AM peptide, or comprising a compound selected from formula XIV through formula XVII (or, more particularly, formula XIV' through XVII'), in association with *(e.g.,* bound to) a GRP peptide. The composition may be in an animal, such as a mammal *(e.g.,* following the administration of the compound to the animal *in vivo*), or it may be *in vitro* (not in an animal).

In another embodiment, the invention relates to a composition comprising a compound selected from formula I through VIII, XII, or XIII (or, more particularly, formula I' through formula XIII'), in association with *(e.g.,* bound to) a blocking antibody of AM, or comprising a compound selected from formula XIV to formula XVII (or, more particularly, formula XIV' through XVII'), in association with *(e.g.,* bound to) a blocking antibody of GRP. The composition may be in an animal, such as a mammal, or it may be *in vitro* (not in an animal).

In another embodiment, the invention relates to a pharmaceutical composition, comprising a compound selected from formula I through VIII, or formula XII through XVII (or, more particularly, formula I' through XVII') and a pharmaceutically acceptable carrier.

In another embodiment, the invention relates to a method (*e.g*., a diagnostic method) for detecting an AM peptide, comprising contacting a sample suspected of containing the peptide with one or more detectably labeled compounds selected from formula I through VIII, XII, or XIII (or, more particularly, formula I'through formula XIII'), and detecting labeled compound that is associated with (bound to) the peptide; or for detecting a GRP peptide, comprising contacting a sample suspected of containing the peptide with one or more detectably labeled compounds selected-from formula XIV through formula XVII (or, more particularly, formula XIV' through formula XVII'), and detecting labeled compound that is associated with (bound to) the peptide. The detection method may be performed *in vivo* or *in vitro.* Optionally, for example when the detection is performed *in vivo*, the detectably labeled compound(s) may be in the form of a pharmaceutical composition.

In other embodiments, the invention relates to kits suitable for treating subjects in need of such treatment. In one embodiment, the kit is suitable for treating a subject suffering from a condition mediated by aberrant expression and/or activity of adrenomedullin (AM); and it comprises one or more of the compounds selected from formula I to VIII, XII, or XIII (or, more particularly, formulas I'through XIII'), or a pharmaceutical composition comprising said compound(s) and a pharmaceutically acceptable carrier and, optionally, a packaging material. In another embodiment, the kit is suitable for treating a subject suffering from a condition mediated by aberrant expression and/or activity of gastrin releasing peptide (GRP); and it comprises one or more of the compounds selected from formula XIV to formula XVII (or, more particularly, formula XIV' through XVII'), or a pharmaceutical composition comprising said compound(s) and a pharmaceutically acceptable carrier and, optionally, a packaging material.

In other embodiments, the invention relates to kits suitable for detecting an AM or GRP peptide (e.g., for a diagnostic method). In one embodiment, the kit is suitable for detecting an AM peptide; and it comprises one or more of the compounds selected from formula I to VIII, XII, or XIII (or, more particularly, formula I' through formula XIII'), which is detectably labeled, and, optionally, means to detect the labeled compound associated with (bound to) the peptide. In another embodiment, the kit is suitable for detecting a GRP peptide; and it comprises one or more of the compounds of formula XIV to formula XVII (or, more particularly, formula XIV' through XVII'), which is detectably labeled, and, optionally, means detect the labeled compound associated with (bound to) the peptide. Kits suitable for *in vivo* detection may further comprise a pharmaceutically acceptable carrier.

In another embodiment, the invention relates to a method for inhibiting GRP-mediated angiogenesis in a subject in need of such treatment, comprising administering to the subject an effective amount of an agent that inhibits expression and/or an activity of GRP (e.g., a compound of one of formula XIV - XVI', or a compound of formula XIV' - XVI'), provided that the GRP-mediated angiogenesis is not angiogenesis involved in tumor growth or metastasis; or a method for preventing or treating condition mediated by GRP-mediated angiogenesis in a subject in need of such treatment, comprising administering to the subject an effective amount of an agent that inhibits expression and/or an activity of GRP (*e.g*., a compound of formula XIV - XVI, or a compound of formula XIV'-XVT), provided that the condition is not cancer.

Conditions that may be treated with such GRP inhibitors include angiogenesis-mediated conditions (conditions mediated by excessive amounts (pathogenic amounts) of angiogenesis), *e.g.,* arthritis; psoriasis; benign growths caused by rapidly dividing cells; brain ischaemia; vascular diseases; ocular diseases *(e.g.,* diabetic retinopathy); fibrosis; deep venous thrombosis; endometriosis; wrinkles; etc. a more extensive disclosure of suitable conditions is presented elsewhere herein. The term "a GRP inhibitor" or "an AM inhibitor," as used herein, refers to an agent which inhibits the expression and/or an activity of GRP or AM, respectively.

In another embodiment, the invention relates to a method for inhibiting angiogenesis-mediated tumor growth in a subject in need of such treatment, comprising administering to the subject an effect amount of an agent that inhibits expression and/or an activity of GRP ((*e.g*., a compound of formula XV, or a compound of formula XV') and detecting or monitoring the reduction in blood vessels (inhibition of angiogenesis).

As noted above, the inventors have developed a two-step screening method to identify agents which modulate an activity of, *e.g.,* a peptide hormone. As used herein, the singular forms "a," "an," and "the" include plural referents unless the context clearly dictates otherwise. For example, an agent of the invention that modulates "an" activity of a peptide hormone of interest may modulate one or more such activities.

In the experiments reported herein, modulatory agents were identified for AM and GRP.

As a starting point, molecules of the NCI small molecule (non-peptide) library were screened. This library contains about 5x10⁶ molecules, which are organized into 2,000 families grouped under the criterion of chemical similarity (Voigt et al. (2001) J. Chem. Inf. Comput. Sci. 41, 702-712). Structures of the compounds are available at the web site cactus.nci.nih.gov/ncidb2. Any library of small organic or inorganic molecules, such as molecules obtained from combinatorial and natural product libraries, can be tested and identified by the methods of the invention.

Furthermore, other types of molecules can also be screened, including (1) peptides, such as soluble peptides, including Ig-tailed fusion peptides and members of random peptide libraries, such as is described in Lam et al. (1991) Nature 354, 82-84; Houghten et al. (1991) Nature 354, 84-86), and combinatorial chemistry-derived molecular libraries made of D- and/or L-configuration amino acids, and specific derivatives of peptides of interest; (2) antibodies *(e.g.,* polyclonal, monoclonal, humanized, anti-idiotype, chimeric, and single chain antibodies as well as Fab, F(ab')₂, Fab expression library fragments, and epitope-binding fragments of antibodies); and (3) phosphopeptides, such as members of random and partially degenerate, directed phosphopeptide libraries, *e.g.,* as in Songyang et al. (1993) Cell 72, 767-778.

The method of the invention was designed based on the assumption that a neutralizing antibody, such as a monoclonal antibody, binds to an epitope on the peptide that is important, if not critical, for receptor recognition. Without wishing to be bound by any particular mechanism, the inventors appear to have confirmed this assumption by the identification of biologically active compounds capable of modulating the physiology of AM and GRP. In addition, the antibody-based colorimetric screening procedure allows for high throughput formats able to analyze thousands of compounds (or more) in very short periods of time.

The first step in the procedure exemplified herein was to identify compounds that interfered with binding between the peptide and its blocking monoclonal antibody. Some details of this first step in the assay are presented in Example 3. See also the results shown in Fig. 1A. In many cases, active compounds could be identified by the naked eye, even before colorimetric quantification (see Fig. 1B). 2,000 parental compounds of the library were screened using this methodology for AM, and 121 of them caused a significant inhibition in color intensity in a statistically significant fashion.

The inventors also screened the same compounds with a blocking monoclonal antibody against GRP (Chaudhry et al. (1999) Clin. Cancer Res. 5, 3385-3393), a peptide similar to AM in size and in chemical characteristics. This allowed the evaluation of the specificity of this methodology, as well as the identification of modulatory agents for GRP. Screening the same clinical library, 109 compounds were identified that inhibited color formation to a significant degree. Only 5 of them were also present among the molecules able to interfere with AM, indicating that, in fact, different combinations of peptide-antibody complexes pulled out distinct sets of small molecules. This clearly shows that this methodology is able to discriminate between target molecules.

As is discussed below, many of the compounds identified in this first step of the assay were not useful for modulating receptor-mediated responses. In the experiments reported herein, only 19.8% of the compounds tested for AM, and 4.6% of the compounds tested for GRP, fulfilled this criterion. Nevertheless, this first step allowed for a rapid primary screening of a large number of compounds, and reduced considerably the number of compounds that must be tested with the more expensive and time-consuming cell-based screen.

Since the first step of the screening strategy was based on the ability of the test molecules to interfere with the binding between the peptide and its antibody, it was possible that not all these molecules would also modify the binding between the peptide and its receptor, even though the monoclonal antibodies used were shown to be neutralizing. To investigate functional consequences of the molecules identified in the-first-step of the screen, all the "positive" compounds were subjected to an analysis of their ability to modify the production of the intracellular second messenger elicited by the specific receptor system.

All the compounds chosen with the primary AM screening were analyzed with a cAMP assay. Details of this second step of the assay are present in Example 4a. From the initial 121 compounds, 24 were able to significantly modulate the amount of cAMP induced by 100 nM AM in Rat2 cells, whereas the other 97 did not modify the cAMP response to AM. Interestingly, some of these compounds reduced the cAMP levels (acted as antagonists) whereas others actually elevated intracellular cAMP levels over the levels induced by AM alone, identifying them as superagonists (Fig. 2A, Table 1). In the absence of AM, none of the compounds elicited any response (Fig. 2A), suggesting that the mechanism of action includes binding of the small molecule to AM rather than to the receptor. That is, the molecules acted as superagonists rather than as agonists. These responses were dose-dependent, with drug responses seen with chemical concentrations as low as 10 nM (Fig. 2B).

**Table 1**

| Compounds that induced consistent effects on modulating second messenger activation by AM or GRP. Some of them were tested for biological activity (4^{th} column). | | | |
|---|---|---|---|
| **Peptide** | **Action on second messengers** | **Code¹** | **Biological activity** |
| AM | Antagonists | 16311 (compound I') | Elevates blood pressure |
| | | 37133 (compound II') | |
| | | 48747 (compound III') | Elevates blood pressure |
| | | 89435 (compound IV') | Elevates blood pressure |
| | | 28086 (compound V') | |
| | | 79422 (compound VI') | |
| AM | Antagonists | 50161 (compound VII') | |
| | Superagonists | 697165 (compound VIII') | |
| | | 697162 (compound IX') | |
| | | 697168 (compound X') | |
| | | 697169 (compound XI') | |
| | | 128911 (compound XII') | Reduces blood pressure |
| | | 145425 (compound XIII') | Reduces blood pressure |
| GRP | Antagonists | 54671 (compound XIV') | |
| | | 77427 (compound XV') | Inhibits cord formation |
| | | 112200 (compound XVI') | |
| | Superagonist | 372874 (compound XVII') | |

The primary difference between AM and CGRP receptors is the nature of the particular RAMP that is associated to CRLR. When the active compounds for AM were added to a CGRP receptor-containing cell in the presence of synthetic CGRP, no effect was observed (Fig. 2D), demonstrating the specificity of these compounds for the binding between AM and its receptor.

For the compounds that showed promising behavior by both screening steps, close structurally related chemical family members were also evaluated. The prediction was that a similar chemical structure would exhibit similar biological behavior. To test this, the following related family members were tested: original compound VIII' and related compounds IX', X' and XI'. In most cases, this analysis produced compounds with stronger activity than the original substance (Fig. 2C), suggesting that grouping compounds based on their chemical similarity could be useful to predict their potential biological activity. That is, one could use a modular approach to the screening process, beginning with the leading 2,000 compounds and then with other members of the promising families. This strategy, combined with the high throughput antibody-based primary screening, allowed for a complete preliminary search of the whole library in a matter of days.

In a similar approach, the small molecules that were identified in the first screening step with the GRP antibody were characterized by their ability to modify IP₃ or Ca²⁺ levels induced by synthetic GRP in cells containing its receptor (see Example 4b and Fig. 3). Again, both antagonist and superagonist molecules were identified. As was the case with modulators of AM, the GRP-interfering small molecules by themselves did not produce any change in IP₃ levels (Fig. 3A). In the Ca²⁺ assay, 1 nM GRP produced a marked elevation of intracellular Ca²⁺ in H1299 cells (Fig. 3B), but pre-exposure of the cells to the identified antagonists greatly reduced the Ca²⁺ spike amplitude (Fig. 3C). The compounds that showed a consistent behavior with either the AM or the GRP systems are summarized in Table 1.

To validate the biological activity of some of the small molecules selected above, several assays were performed. For example, an important function of AM is the regulation of blood pressure. As is described in more detail in Example 5, injection of screen-selected AM superagonists (at 20 nmols/Kg) in hypertensive rats induced a profound and long-lasting decrease from basal levels in blood pressure ranging from 50 to 70 mm Hg (Fig. 4A,B). Vehicle alone (DMSO in PBS) at the same concentration did not alter blood pressure (Fig. 4A). On the other hand, when screen-selected small molecule AM antagonists were injected into normotensive animals, also at 20 nmols/Kg, an elevation in blood pressure was observed (Fig. 4C). The blood pressure profile generated by the superagonists was similar to the one elicited by the peptide itself (that was used as a control in Fig. 4B), suggesting that these small molecules may be enhancing the effect of circulating AM. Similar *in* vivo effects are expected for the remaining modulators of AM. Further studies to elucidate the mechanism of action of AM modulators are presented in Example 11.

Biological activity of small molecule GRP antagonists is also demonstrated in the Examples. The influence of some of the GRP antagonists was analyzed in angiogenic models. Example 6 demonstrates that GRP can induce cord formation *in vitro,* in a culture of endothelial cells grown on Matrigel, and that a small molecule GRP inhibitor of the invention greatly reduces the complexity of the tubular lattice. Example 7 demonstrates, in an in *vivo* model of directed angiogenesis (DIVAA), that GRP exhibits angiogenesis potential *in vivo,* and that this angiogenesis is inhibited by a small molecule GRP antagonist of the invention. Growth inhibition assays show that a small molecule GRP inhibitor inhibits the growth of a lung cancer cell line *in vitro* (Example 8), and that it reduces the number of colonies in a clonogenic assay (Example 9). The inhibitors also display inhibitory activity in an *in vivo* assay of tumor growth in mice (Example 10). The experiments in these examples were carried out primarily with the small molecule GRP inhibitor, compound XV' (77427). Similar effects are expected for the remaining antagonists of GRP.

In a preliminary analysis, the inventors have identified some elements that appear to be conserved among some of the modulatory agents identified herein. Without wishing to be bound by any particular model, it is suggested that the modulatory agents fall into several "families" of structures. A careful analysis of the chemical structures of some of the active compounds for AM reveals some common characteristics. The most active antagonists (*e.g.,* compounds of formula I' (16311), formula IV' (89435) and formula VIT' (50161)) have in common an aromatic ring separated from a three-substituted nitrogen by 4 elements. There is also a hydroxy group at 2 or 3 elements from the nitrogen. Several compounds with superagonist activity (e.g., compounds of formula XIII' (145425), formula XII' (128911) and formula VIII' (697165)) share the presence of nitrogenated heterocycles with oxygen atoms at similar distances. Nevertheless, the surprising simplicity of compound XII' (128911) suggests that the activity may be due just to the presence of a nitrogen with sp² hybridization situated at a determined distance from the oxygen.

The resolution of the three-dimensional structures of the AM-AM receptor complex and the GRP-GRP receptor complexes should allow one to identify more precisely the binding sites of the small molecules identified herein and to introduce direct design modifications of these molecules to fit the active site more closely. Such methods are conventional. See, *e.g.*, rational design methods in Ghosh et al. (2001) Curr. Cancer Drug Targets 1, 129-140. Additional optimization can be obtained by generating additional compounds by combinatorial chemistry, for example by modifying slightly the chemical backbone identified here with different radicals. Such methods are conventional See, *e.g.,* Gray et al. (1998) Science 281, 533-538 and Poyner et al. (2002) Pharmacol Rev 54, 233-246.

Also disclosed is a method to identify an agent that modulates (*e.g*., modulates an activity of) a peptide which interacts specifically with a receptor, such as a peptide hormone, preferably AM or GRP, comprising
a) contacting the peptide, a blocking antibody of the peptide, and a putative binding-inhibitory agent,
b) detecting binding of the peptide to the anybody, and
c) selecting an agent which inhibits (*e.g*., disrupts) said binding, compared to the binding

in the absence of the putative binding-inhibitory agent, thereby identifying a binding-inhibitory agent.

The binding-inhibitory agent may be an antagonist or an agonist of the peptide. Preferably, the putative-binding-inhibitory agent is a non-peptide small molecule. In one embodiment, the method is a high throughput method (assay).

In a preferred embodiment, the above method further comprises
d) contacting a binding-inhibitory agent identified as above, the peptide, and a cell that comprises a receptor for the peptide,
e) detecting the amount in the cell of a second messenger induced by the peptide, and
f) selecting an agent that modulates the amount of the second messenger in the cell,
compared to the amount in the cell in the absence of the agent, thereby identifying a modulatory agent.

The modulatory agent may be an antagonist or an agonist (*e.g*., a superagonist) of the peptide. For example, the agent may be an agonist or antagonist of an activity of the peptide, such as its binding to a receptor, the stimulation (expression) of a second messenger, or any of the other activities described elsewhere herein. In one embodiment, the cell comprises a receptor for AM, and the second messenger is AM-induced cAMP. In another embodiment, the cell comprises a receptor for gastrin releasing hormone (GRP), and the second messenger is GRP-induced IP₃ or Ca⁺⁺. Preferably, the putative binding-inhibitory agent is a non-peptide small molecule. In one embodiment, the method is a high throughput method (assay).

Any of the preceding methods for identifying putative modulatory agents may further comprise additional steps, some of which are discussed elsewhere herein. The invention also relates to modulatory agents which are identified and/or characterized by a method of the invention, particularly small, non-peptide, molecules that are encompassed by one of the generic structures identified herein.

Modulatory agents of the invention (*e.g*., small molecule non-peptide compounds) can be prepared (e.g., synthesized) fully conventionally, using known reaction chemistry, starting from known materials or materials conventionally preparable. Procedures for synthesizing small molecule, non-peptide, compounds can readily produce gram amounts of a compound of interest. Many compounds of the invention are readily available from standard sources, such as chemical supply houses, or can be generated from commercially available compounds by routine modifications.

The present invention also relates to useful forms of the compounds as disclosed herein, such as pharmaceutically acceptable salts and prodrugs of all the compounds of the present invention. Pharmaceutically acceptable salts include those obtained by reacting the main compound, functioning as a base, with an inorganic or organic acid to form a salt, for example, salts of hydrochloric acid, sulfuric acid, phosphoric acid, methane sulfuric acid, camphor sulfonic acid, oxalic acid, maleic acid, succinic acid and citric acid. Pharmaceutically acceptable salts also include those in which the main compound functions as an acid and is reacted with an appropriate base to form, *e.g.*, sodium, potassium, calcium, magnesium, ammonium, and chlorine salts. Those skilled in the art will further recognize that acid addition salts of the claimed compounds may be prepared by reaction of the compounds with the appropriate inorganic or organic acid via any of a number of known methods. Alternatively, alkali and alkaline earth metal salts are prepared by reacting the compounds of the invention with the appropriate base via a variety of known methods..

The following are further examples of acid salts that can be obtained by reaction with inorganic or organic acids: acetates, adipates, alginates, citrates, aspartates, benzoates, benzenesulfonates, bisulfates, butyrates, camphorates, digluconates, cyclopentanepropionates, dodecylsulfates, ethanesulfonates, glucoheptanoates, glycerophosphates, hemisulfates, heptanoates, hexanoates, fumarates, hydrobromides, hydroiodides, 2-hydroxy-ethanesulfonates, lactates, maleates, methanesulfonates, nicotinates, 2-naphthalenesulfonates, oxalates, palmoates, pectinates, persulfates, 3-phenylpropiionates, picrates, pivalates, propionates, succinates, tartrates, thiocyannates, tosylates, mesylates and undecanoates.

Preferably, the salts formed are pharmaceutically acceptable for administration to mammals. However, pharmaceutically unacceptable salts of the compounds are suitable as intermediates, for example, for isolating the compound as a salt and then converting the salt back to the free base compound by treatment with an alkaline reagent. The free base can then, if desired, be converted to a pharmaceutically acceptable acid addition salt. oxygen.

Agents of the invention may be used in therapeutic methods for conditions (including pathogenic conditions, or diseases) that are mediated by aberrant expression and/or activity of a peptide hormone, such as AM or GRP, and/or for conditions (including non-pathogenic conditions) that respond to an increase or decrease in the expression or activity of the peptide hormone. The term "aberrant" expression and/or activity, as used herein, includes expression or activity that is higher or lower than a base line value, such as the amount present in a subject who does not exhibit symptoms of the condition, or who does not exhibit a predisposition to the condition. The expression or activity may be an "under" -expression or -activity, or an "over" - expression or -activity. When aberrant expression results in undesirable symptoms, the condition is sometimes said to be a pathological condition.

The therapeutic methods include diagnosis, treatment, prevention, and/or amelioration of symptoms of any of a variety of conditions (e.g., pathological conditions) in a subject, or modulation of physiological conditions (e.g., non-pathological conditions, such as the stimulation or inhibition of appetite), which are associated with AM or GRP activity. The subject (*e.g*., a patient) can be any suitable animal, including mammals, birds, reptiles, fish, amphibians, etc. Suitable subjects include, *e.g.*, experimental animals (such as mice, rats, guinea pigs, rabbits, fish, frogs, etc.), pets, farm animals (such as cows, pigs, horses, birds such as chickens or geese, etc.), and primates, especially humans.

With regard to agents that modulate AM activity, AM levels are dysregulated in many pathologies (e.g., in humans), such as hypertension, heart failure, sepsis, cancer, or diabetes, when compared to healthy controls. See, e.g., the AM-mediated conditions discussed in U.S. patent application 20020055615. This correlation, together with experimental actions of AM in relevant model systems, implicates this molecule in the pathophysiology of such conditions. Interestingly, changes in AM levels may have apparently paradoxical effects on a patient's health, depending on the particular disease studied.

For example, elevated AM expression seems to exert a protective role in renal and cardiovascular diseases, sepsis, and in central nervous system ischemia. Without wishing to be bound by any particular mechanism, it is suggested that overexpression of AM is protective due to its vasodilator activity. An agent that acts as an agonist or superagonist of AM can be used to treat or prevent conditions that are ameliorated by the expression of AM, such as, e.g., vascular diseases, trauma, malignant hypotension, catecholamine disorders, or the other conditions noted above.

In other circumstances, elevated AM expression appears to worsen a pathological condition, such as the progression of type 2 diabetes and cancer. In diabetic rats, injection of AM results in a reduction of circulating insulin levels and a concomitant hyperglycemia, whereas application of a monoclonal antibody against AM lowers glucose levels and ameliorates postprandial hyperglycemia. AM antagonists of the invention may be used to treat diabetes, e.g., by regulating insulin secretion and/or blood glucose metabolism. In cancer cells, AM acts as a tumor survival factor. This tumor survival may be influenced by various activities of AM, such as elevation of tumor cell growth, circumventing apoptosis, increasing migration, and enhancement of angiogenesis. Among the types of neoplastic transformation (e.g., cancerous cells) that can be treated by AM antagonists of the invention are, *e.g.*, adrenal, nervous system (*e.g.,* (*e.g.,* brain tumors, such as gliomas, astrocytomas or neuroblastomas), renal, lung (e.g., small cell lung cancer), pancreatic, gastric, gastrointestinal, lung (*e.g*., small cell lung cancer), colon, colorectal, prostate, ovarian and breast cancerous cells, and chondrosarcoma, as well as other types of neoplastic diseases discussed herein with reference to GRP antagonists. An agent that acts as an antagonist of AM can be used to treat or prevent conditions that are rendered worse by the expression of AM, such as the conditions noted above, or others.

Additional conditions that can be diagnosed, treated, and/or prevented with antagonists or agonists (*e.g*., superagonists) of AM will be evident to the skilled worker. Among the physiological effects of AM are bronchodilation, regulation of hormone secretion, neurotransmission, antimicrobial activities, and regulation of cell growth and migration. One of skill in the art will recognize a variety of conditions that are mediated by these, or other, effects. Among the treatment methods for which agents of the invention are suitable are, *e.g.*, treating conditions related to pregnancy (*e.g*., diagnosing and/or treating preeclampsia or promoting fetal growth); regulating activity in areas of the central nervous system, (e.g., regulation of neurotransmission or neuron growth, such as in, e.g., Alzheimer's disease); lessening or inhibiting the allergic response due to the degranulation of mast cells; treating bacterial and fungal infections by inhibiting or preventing bacterial or fungal growth; facilitating the healing of chafed skin, skin lesions, wound repair, and surgical incisions (*e.g*., by applying to the surface of the skin of a subject an amount of one or more of the agents of the present invention effective to facilitate healing); and promoting organ and bone development.

For a further discussion of some conditions that can be diagnosed, treated and/or prevented with AM antagonists or agonists, and suitable methods that can be applied to use of the modulatory compounds of the invention, see U.S. patent application 20020055615 (Cuttitta et al.).

With regard to agents that modulate GRP activity, GRP levels are dysregulated in many pathologies (e.g., in humans), such as cancers, compared to healthy controls. The inventors have used a neutralizing monoclonal antibody against GRP in phase I/II clinical trials of previously treated small cell lung cancer patients (Chaudhry et al. (1999) Clin. Cancer Res. 5, 3385-3393). The results of that trial, including a curative complete response, suggest that inhibitors of GRP biology may be very useful in addressing clinical problems.

Several endocrine peptides have been shown to promote angiogenesis. Here, the inventors demonstrate that GRP is another endocrine peptide which promotes angiogenesis (is a pro-angiogenic factor). Angiogenesis is a complex process that requires endothelial cell growth and migration, extracellular matrix remodeling, formation of tubular structures, and loop formation, among other mechanisms. The studies reported in Example 6 show the ability of an exemplary small molecule to interfere with the cord formation ability of GRP. GRP by itself promoted the development of a complex meshwork made of pseudo-capillaries. The simultaneous application of compound XV' (77427) resulted in a marked decrease in the complexity of the tubular network, indicating a utility of this compound in antiangiogenic interventions. Example 7 shows by an *in vivo* assay that GRP exhibits angiogenic potential *in vivo,* and that this angiogenesis can be inhibited by compound XV'. That is, GRP is a potent angiogenic factor, which acts directly to stimulate angiogenesis (rather than through intermediate effects, such as the stimulation of angiogenic factors such as VEGF or bFGF). Example 10 shows that an exemplary GRP antagonist of the invention (compound XV') effectively inhibits tumor-induced angiogenesis in an *in vivo* assay. In the experiments described in this example, a human tumor cell line is transplanted into a mouse (as a xenograft), and the small molecule antagonist effectively blocks tumor growth.

The demonstrations herein that GRP is a pro-angiogenic factor, and that at least two types of GRP inhibitors - small (non-peptide) molecules and monoclonal antibodies - can inhibit angiogenesis, suggests that any of a broad genus of types of GRP inhibitors can be used to inhibit angiogenesis. Inhibitors of expression of GRP (*e.g.*, antisense molecules, siRNAs, etc) or inhibitors of GRP expression (*e.g*., antibodies, such as monoclonal antibodies specific for GRP, or small molecule, non-peptide, antagonists of GRP) can be used. Preferably, the small molecule inhibitor is a compound of formula XV or XV'.

Among the many types of angiogenesis-mediated conditions (conditions mediated by aberrant angiogenesis) which can be treated with GRP inhibitors are, *e.g.,* arthritis (*e.g*, rheumatoid arthritis); psoriasis; benign growths caused by rapidly dividing cells (*e.g.,* noncancerous melanomas); brain ischaemia; vascular diseases (*e.g*. atherosclerosis, myocardial angiogenesis, post-balloon angioplasty, vascular restenosis, neointima formation following vascular trauma, vascular graft restenosis, coronary collateral formation, deep venous thrombosis, ischemic limb angiogenesis); ocular diseases involving ocular neovascularization or related ocular diseases and disorders (*e.g*., diabetic neovascularization, neovascular glaucoma, macular degeneration, diabetic and other retinopathy, retrolental fibroplasia and corneal diseases); fibrosis (*e.g.*, fibrosis associated with a chronic inflammatory condition, lung fibrosis, chemotherapy-induced fibrosis, wound healing (*e.g.*, of chronic wounds) with scarring and fibrosis; deep venous thrombosis; endometriosis; wrinkles (*e.g.*, UVB-induced wrinkles), etc. In general, anti-angiogenic agents of the invention may be used to treat any disease or condition in which angiogenesis or cell migration/invasiveness is pathogenic. In some embodiments, the angiogenesis-mediated condition is not tumor growth.

The studies shown in Examples 6 through 10 are performed with an exemplary GRP antagonist of the invention (a compound of formula XV'). The other GRP antagonists of the invention are also expected to exhibit similar effects.

One embodiment of the invention is a method for inhibiting angiogenesis-mediated tumor growth in a subject in need of such treatment, comprising administering to the subject an effective amount of an agent that inhibits the expression and/or an activity of GRP (*e.g.* a compound of the invention). In some embodiments, *e.g.*, when the GRP inhibitor is a compound of formula XV or XV', an additional step is added which reflects the ability of GRP inhibitors to inhibit angiogenesis (*e.g*., detecting or monitoring the reduction in blood vessels (inhibition of angiogenesis)).

A variety of types of neoplastic diseases (*i.e.,* cellular proliferative diseases) can be treated with GRP antagonists (*e.g.*, tumor growth can be reduced). Among the proliferative conditions that benefit from administration of the agents of the invention are, e.g., sarcomas, carcinomas, lymphomas, malignant melanomas, and benign growths caused by rapidly dividing cells. The disease or condition being treated may be primary tumor growth, tumor invasion or metastasis. Cancers of the types discussed above with regard to AM antagonists, *e.g.,* adrenal, nervous system (*e.g.*, brain tumors, such as gliomas, astrocytomas or neuroblastomas), renal, lung, pancreatic, gastric, gastrointestinal, lung, colon, colorectal, prostate, ovarian and breast cancerous cells, and chondrosarcoma, are included.

Conditions which are mediated by an under-expression of a GRP-mediated condition can also be treated with agents of the invention. That is, GRP agonists (*e.g.*, superagonists) are useful for treating conditions in which *increased* angiogenesis is desirable. Such conditions include, e.g., coronary or peripheral artery disease; any form of tissue ischemia resulting from vascular occlusion, vascular disease or surgery (*e.g*., peripheral limb ischemia or hepatic arterial occlusion in liver transplantation); organ or tissue transplantation (*e.g*., liver organogenesis, or in conjunction with cellular therapy and transplantation of pancreatic islet cells in the treatment of diabetes, as vascular endothelium acts to stimulate or induce pancreatic organogenesis and insulin production by pancreatic beta cells); and acceleration or enhancing of fracture repair or wound healing (including recovery from surgical wounds, and treatment of chronic wounds with scarring and fibrosis).

GRP is involved in a number of other physiological functions, which will be evident to a skilled worker. These functions include, *e.g.*, the suppression of food intake, regulation of glucose homeostasis, regulation of short-term memory, and enhancement of hypotension. Among the conditions that can be treated or prevented with GRP antagonists are, *e.g.,* eating disorders (such as anorexia or bulimia, in which stimulation of food intake is desirable) and low blood pressure (hypotension). GRP antagonists can also be used commercially when it is desirable to increase the weight of animals designated for meat production, such as cows or pigs. GRP antagonists can also be used to improve breathing in premature babies (bronchopulmonary dysplasia), or to treat gastrointestinal disorders, such as peptic ulcer and pancreatitis. Among the conditions which can be treated or prevented with GRP agonists (*e.g.*, superagonists) are, *e.g.,* obesity, diabetes or hypertension. Other conditions suitable for treatment with GRP antagonists or agonists will be evident to the skilled worker. For a discussion of some physiological functions of GRP, and some disease conditions that can be treated or prevented with antagonists or agonists of GRP, see, *e.g.,* Mantey et al. (2001) The Journal of Biological Chemistry 276, 9219-9229; Merali et al. (1999) Neuropeptides 33, 376-386; and Ohki-Hamazaki et al. (1997) Nature 390, 165-169.

Any of the suggested treatment or prevention methods using AM or GRP antagonists or agonists (*e.g.*, superagonists) may be combined with other therapeutic modalities, and combinations of the agents of the invention may be used.

In some of the inventive methods for modulating an activity of a peptide, or for detecting a peptide, the peptide is "contacted" with a modulatory agent of the invention. This contacting may be achieved in a subject (*in vivo*) or outside of an animal (*in vitro*)*.* Suitable methods for contacting are conventional and well-known in the art. For example, a peptide can be contacted with a compound in a cell (either in *vivo or in vitro*) by introducing the compound by injection, such as microinjection, electroporation, sonoporation, a gene gun, liposome delivery (*e.g.*, Lipofectin^{®}, Lipofectamine^{®} (GIBCO-BRL, Inc., Gaithersburg, MD), Superfect^{®} (Qiagen, Inc. Hilden, Germany) and Transfectam^{®} (Promega Biotec, Inc., Madison, WI), or other liposomes developed according to procedures standard in the art), or receptor-mediated uptake and other endocytosis mechanisms.

In methods of treatment according to the invention, an effective amount of an agent of the invention is administered to a subject. The term "an effective amount," as used herein, means an amount that elicits a detectable response (*e.g*., amelioration of a symptom or a physiological response); the degree of the response can be minimal, provided that it is detectable. Similarly, in methods for modulating an activity of a peptide, an effective amount of an agent of the invention is contacted with the peptide. An "effective amount" in this context means an amount that elicits a detectably amount of modulation.

In methods of treatment, the agent can be administered (delivered) by any of a variety of conventional procedures. Suitable routes of administration include parenteral and non-parenteral routes. Parenteral routes include, *e.g.*, intravenous, intraarterial, intraportal, intramuscular, subcutaneous, intraperitoneal; intraspinal, intrathecal, intracerebroventricular, intracranial, intrapleural or other routes of injection. Non-parenteral routes include, e.g., oral, nasal, transdermal, pulmonary, rectal, buccal, vaginal, ocular. Topical administration is desirable, for example, when the condition to be treated is presented on an accessible surface, such as a mucosal surface. Topical administration to the skin (cutaneous delivery) is particularly useful for the treatment of, *e.g.*, psoriasis or skin cancer. In a preferred embodiment, the administration is timed, slow-release, aerosolized administration. Administration may also be by continuous infusion, local administration, "directed systemic" administration, sustained release from implants (gels, membranes or the like), and/or intravenous injection.

Dosages to be administered can be determined by conventional procedures known to those of skill in the art. See, *e.g.,* The Pharmacological Basis of Therapeutics, Goodman and Gilman, eds., Macmillan Publishing Co., New York. The dosage should not be so large as to cause adverse side effects, such as unwanted cross-reactions, anaphylactic reactions, and the like. Factors to be considered include the activity of the specific therapeutic agent involved, its metabolic stability and length of action, mode and time of administration, drug combination, rate of excretion, the species being treated, and the age, body weight, general health, sex, diet, and severity of the particular disease-states of the host undergoing therapy. Dosages can be selected in a manner customary for treatment with comparable agents for the same condition.

The agents of the invention may be formulated as pharmaceutical compositions, with any of a variety of conventional, pharmaceutically acceptable carriers, diluents and/or excipients. For suitable components and methods of preparing pharmaceutical compositions, see, *e.g.*, Remington's Pharmaceutical Sciences, 18th ed., Mack Publishing Company (1990); the Handbook of Pharmaceutical Excipients, American Pharmaceutical Association (current edition); and Pharmaceutical Dosage Forms: Tablets (Lieberman, Lachman and Schwartz, eds., current edition, published by Marcel Dekker, Inc.)

Agents of the invention may also be used in detection (e.g., diagnostic) procedures. For example, compounds of the invention can be labeled with conventional labels, using conventional procedures, and then used to detect AM or GRP, *in vivo* or *in vitro* (ex vivo). Compounds in which a detectable label is present are sometimes referred to herein as "detectably labeled" compounds. Methods (means) of labeling the compounds and detecting the detectable labels (e.g., detecting labeled compounds that have become associated with (e.g., bound to) the peptide) are conventional and well-established. The detection may be direct, or indirect (*e.g.*, as in some enzymatic detection methods). In some embodiments, the detection is quantitative.

With regard to *in vivo* imaging, since both AM and GRP are turned over rapidly in the body, there is little circulating AM or GRP. Thus, *in vivo* detection (imaging) to determine where AM or GRP is localized in an organism can indicate the site at which the peptide is produced. Labeled-compounds of the invention can be used in any situation in which a tracer of AM or GRP is desirable. Suitable labels will be evident to a skilled worker and include, *e.g.*, heavy metals, which can be detected in PET scans, and radioactive labels, such as ¹³¹I or other short-lived radioactive tracers. Because many cancers are associated with the production of large amounts of AM or GRP, detection (diagnostic) methods as above with AM or GRP modulatory compounds are useful for detecting the presence and/or location of a cancer. Other uses of such in *vivo* detection (diagnostic) methods will be evident to the skilled worker.

As for *in vitro* methods (assays), a compound of the invention can be labeled with a conventional detectable label, such as a fluor or an enzyme (*e.g*., lactoperoxidase, alkaline phosphatase, or beta galactosidase), and then contacted with a tissue sample (such as a pathology sample) in order to visualize the presence of the peptide (*e.g*., to identify a cancerous tissue). The compounds of the invention can be used in a variety of *in vitro* assay procedures, not only to detect the presence of a peptide of interest, but also to quantitate the amount of the peptide. For example, the compound can substitute for a monoclonal antibody in a conventional radioimmunoassay. In addition, the modulatory agents can be used for pharmacological drug design. For example, by analyzing the three dimensional structure of a complex between an AM or GRP peptide, or a blocking antibody for the peptide, and a compound identified herein or a variant thereof, using NMR or NMR imaging, one can screen and/or characterize variants that are more effective antagonists or agonists than the starting compound. (The compounds identified herein can serve as comparative controls in such a method.) Other suitable *in vitro* methods in which compounds of the invention can be used will be evident to the skilled worker.

Detection methods of the invention can be used to detect (*e.g.*, diagnose or monitor) any of the conditions described elsewhere herein, or others, which are mediated by aberrant expression and/or activity of AM or GRP. For example, one can monitor a condition (*e.g.,* a disease condition) by measuring the amount of AM or GRP in a sample, wherein the presence of the AM or GRP indicates the existence of, or predisposition to, the condition. Examples of conditions that can be diagnosed or monitored by methods of the invention include, but are not limited to, diabetes; renal diseases, such as severe uremia; bone diseases, such as neoplastic disease; skin diseases; and blood related diseases, such as leukemia.

In view of the tight association of the small molecules of the invention to AM or GRP, or, in some cases, to the receptors for AM or GRP, the small molecules of the invention can also be used to target additional therapeutic agents to cells in need of such treatment (cells which express AM or GRP, or receptors for those peptides). Suitable therapeutic agents (*e.g.*, toxins such as ricin, diphtheria toxin, etc., to target tumor cells) will be evident to the skilled worker. For some examples of suitable therapeutic agents, see, *e.g.*, US application 20020176819 and WO00/54805.

In another aspect of the invention, modulatory compounds of the invention are found in complexes with (or are in compositions with) the AM or GRP peptides, or with blocking antibodies specific for those peptides. In the complexes (or compositions) of the invention, the compounds associate with (*e.g.,* bind to) the peptides or antibodies by any of a variety of means that are well-know to skilled workers. The types of association include, *e.g.,* covalent bonds or non-covalent bonds (*e.g*., passively adsorbed, such as by electrostatic forces, ionic or hydrogen bonds, hydrophilic or hydrophobic interactions, Van der Waals forces, etc.).

Complexes of the invention can provide tools for the characterization of receptors, binding proteins, and other binding sites, and can help elucidate the mechanism of action of the peptide hormones. For example, because the blocking antibodies described herein mimic the receptors to which the peptide hormones bind, the antibodies can serve as surrogate receptors. Thus, small molecule/antibody complexes can serve as artificial ligand/receptor complexes. See also the types of studies described in Poyner et al. (2002) Pharmacol. Rev. 54, 233-246 and Pio et al. (2002) Microsc. Res. Tech. 57, 23-27. In another embodiment, a complex between a peptide or antibody and a compound of the invention can be used to identify and/or characterize other compounds that exhibit more effective antagonist of agonist activity, e.g., as discussed above.

When a modulatory compound of the invention is administered to an animal, a complex of the compound and AM or GRP may form *in vivo* (in the animal).

An *in vitro* complex of a modulatory compound of the invention and a peptide or blocking antibody of the invention is sometimes referred to herein as an "isolated" complex. As used herein, the term "isolated," when referring, e.g., to a complex of the invention; means that the material is not in its naturally occurring form, is generated artificially *in vitro,* and/or is isolated or separated from at least one other component with which it is naturally associated. For example, a naturally-occurring complex as above, when present in its natural living host, is not isolated, but the same complex, separated from some or all of the coexisting materials in the natural system, is isolated. Such complexes could be part of a composition, and still be isolated in that such composition is not part of its natural environment.

Another aspect of the invention is a kit, suitable for performing any of the methods (*e.g.*, assays) of the invention. For example, the kit may be suitable for treating a subject (*e.g*., a subject suffering from a condition mediated by aberrant expression and/or activity of AM or GRP), or for detecting an AM or GRP peptide, *in vitro* or *in vivo.* The components of the kit will vary according to which method is being performed. Generally, a kit of the invention comprises one or more of the compounds of formula I through formula XVII (or, more particularly, I' through XVII'), or a pharmaceutical composition comprising said compound(s) and a pharmaceutically acceptable carrier. The kits also optionally contain means (*e.g*., suitable reagents) for monitoring disease conditions and/or for detecting AM or GRP. Reagents for performing suitable controls may also be included.

Optionally, the kits comprise instructions for performing the method. Kits of the invention may further comprise a support on which a cell can be propagated (*e.g*., a tissue culture vessel) or a support to which a reagent used in the method is immobilized. Other optional elements of a kit of the invention include suitable buffers, media components, or the like; a computer or computer-readable medium for storing and/or evaluating the assay results; logical instructions for practicing the methods described herein; logical instructions for analyzing and/or evaluating the assay results as generated by the methods herein; containers; or packaging materials. The reagents of the kit may be in containers in which the reagents are stable, e.g., in lyophilized form or stabilized liquids. The reagents may also be in single use form, e.g., in single dosage form for use as therapeutics, or in single reaction form for diagnostic use.

Kits of the invention have many uses, which will be evident to the skilled worker. For example, they can be used in experiments to study factors involved receptor-mediated activities; to detect the presence of AM or GRP in a cell or tissue, in *vitro* or *in vivo*; to treat a condition mediated by aberrant expression and/or activity of AM or GRP; to monitor the course of such a treatment; or to identify more effective modulatory agents for AM or GRP. A modulatory agent of interest can be characterized by performing assays with the kit, and comparing the results to those obtained with known agents (or by comparison to a reference). Such assays are useful commercially, *e.g.*, in high-throughput drug studies.

In the foregoing and in the following examples, all temperatures are set forth uncorrected in degrees Celsius; and, unless otherwise indicated, all parts and percentages are by weight.

### EXAMPLES

### Example 1 - Small molecule library

The small molecule repository that the NCI has collected since 1955 was used. This library contains about 500,000 compounds organized in 2,000 families of chemically similar molecules. The construction of the library has been described in Voigt *et al.* (*supra*) and can be viewed at the web site cactus.nci.nih:gov/ncbidb2. All compounds were provided diluted in DMSO.

### Example 2 - Reagents

Synthetic human AM and GRP were purchased from Peninsula (S. Carlos, California). Synthetic CGRP and forskolin were obtained from Sigma (St. Louis, Missouri). Blocking monoclonal antibodies against AM³⁰ and GRP²⁰ were produced in-house and labeled with peroxidase using EZ-Link Plus Activated Peroxidase (Pierce, Rockford, Illinois).

### Example 3 - Primary screening for AM and GRP (step #1 of the assay)

Human synthetic AM was solid-phased into PVC 96-well plates (Fisher Scientific, Pittsburgh, Pennsylvania) by incubating 50 µl of AM (at 1 nmols/µl) per well for 1 h. To solid-phase GRP into the plates, these were previously treated with glutaraldehyde as described (Kasprzyk et al. (1988) Anal. Biochem. 174, 224-234). After discarding the coating solution, the plates were blocked with 200 µl per well of 1% bovine serum albumin (BSA) in phosphate buffer saline (PBS). After 1 h, this solution was aspirated off and 50 µl containing 1 µM of one of the compounds of the library in PBS was added per well. Immediately after, 50 µl of labeled antibody (at 2.4 µg/ml) were added on each well and the solution was allowed to react for 1 h. Following 3 thorough washes with 1% BSA in PBS to remove the unbound antibodies, peroxidase activity was developed using o-phenylenediamine dihydrochloride (Sigma) as a substrate. The reaction product was quantified with a plate reader (Spectra Rainbow, Tecan, Austria) at 450 nm. Each plate contained several internal controls including wells without any coating that are used to calculate non-specific binding; wells where no potential antagonists were added, which provided maximum binding; and wells where the unlabeled antibody (at 1.2 µg/ml) substituted the small molecule, as a positive inhibition control (Fig. 1B). Each compound was added to duplicate wells in the same plate. A positive hit was defined as a compound that was able to significantly reduce the amount of reaction product in three independent plates.

### Example 4 - Analysis of second messengers (step #2 of the assay)

### a. cAMP analysis for AM and CGRP

The fibroblast cell line Rat2 has been shown to contain specific AM receptors and react to AM addition by elevating its intracellular cAMP contents. This cell line was obtained from the American Tissue Culture Collection (ATCC, Manassas, Virginia) and maintained in RPMI-1640 supplemented with 10% fetal bovine serum (FBS, Invitrogen, Carlsbad, California). Cells were seeded in 24-well plates at 2 x 10⁴ cells/well and incubated at 37 °C in 5% CO₂ until they reached 80% confluency. Before the assay, cells were incubated for 15 min in TIS medium (RPMI-1640 plus 10 µg/ml transferrin, 10 µg/ml insulin, and 50 nM sodium selenite) containing 1% BSA, 1 mg/ml bacitracin, and 100 µM isobutylmethylxanthine. Peptides and small molecules were applied in the same medium for 5 min at the indicated concentrations in a volume of 250 µl. The reaction was terminated by adding an equal volume of ice-cold ethanol. cAMP contents were measured using the Biotrac cAMP radioimmunoassay (Amersham Biosciences, Piscataway, New Jersey), as described (Pio et al. (2001) J. Biol. Chem. 276, 12292-12300).

A cell line expressing the CGRP receptor was generated by transfecting HEK 293 cells with CRLR and RAMP1 (a generous gift from Dr Debbie Hay, Hammersmith Hospital, London, UK). The analysis was performed as above, but using CGRP instead of AM as the main agonist. In both cases, forskolin was used as a positive control at 50 µM.

Details of the above analyses are discussed in the Brief Description of Figure 2, and results of the analyses are shown in Figure 2.

### b. IP₃ and Ca²⁺ analysis for GRP

The lung cancer cell line H-1299 has been shown to contain specific GRP receptors. This cell line was obtained from ATCC and cultured as the other cell lines. The signal transduction pathway for GRP includes elevation of intracellular levels of IP₃ and Ca²⁺ and these were investigated as previously shown (Ryan et al. (1998) J. Biol. Chem, 273, 13613-13624). Briefly, to quantify IP₃, contents cells were subcultured into 24-well plates (5 x 10⁴ cells/well). After a 24 h incubation period at 37 °C, the cells were incubated with 3 µCi/ml myo-[³H]inositol in growth medium supplemented with 2% FBS for an additional 24 h. Incubation volumes were 500 µl of assay buffer/well containing 135 mM sodium chloride, 20 mM HEPES (pH 7.4), 2 mM calcium chloride, 1.2 mM magnesium sulfate, 1 mM EGTA, 20 mM lithium chloride, 11.1 mM glucose, and 0.05% BSA (v/v) with or without any of the molecules studied at 37 °C for 30 min. Experiments were terminated with 1 ml of ice-cold hydrochloric acid/methanol (0.1%, v/v). [³H]IP₃ was eluted off Dowex AG-1-X8 anion exchange columns with 2 ml of 1 mM ammonium formate and 100 mM formic acid. Each of the eluates was collected and mixed with 10 ml of scintillation mixture (BioSafe, Research Products International Corp, Mount Prospect, Illinois), and the radioactivity was measured in a LS 3801 β counter (Beckman, Somerset, New Jersey).

Calcium levels were analyzed by loading the cells with 2 µM FURA-2/AM (Molecular Probes, Eugene, Oregon) for 30 min at 37 °C. After washing two times with TIS, 2 ml of cell suspension were placed in a Delta PTI Scan 1 spectrofluorimeter (Photon Technology - International, South Brunswick, New Jersey) equipped with a stir bar and water bath (37 °C). Fluorescence was measured at dual excitation wavelengths of 340 nm and 380 nm using an emission wavelength of 510 nm.

Details of the above analyses are discussed in the Brief Description of Figure 3, and results of the analyses are shown in Figure 3.

### Example 5 - Measurement of blood pressure in vivo

AM is a potent and long-lasting vasodilator Therefore it was expected that AM antagonists would elevate blood pressure and AM superagonists would decrease it further. In consequence, suspected antagonists were analyzed in normotensive rats (10-week-old Lewis/ssncr males, SAIC, Frederick, Maryland) and suspected superagonists in hypertensive animals (10-week-old SHR males, Taconic Farms, Germantown, NY).

Animals were anaesthetized with 3% halothane, intubated, and maintained with 1% halothane in 70% nitrous oxide and 30% oxygen (VMS Anesthesia Machine, Matrx, Medical Inc., Orchard Park, New York) at 82 strokes/min. Rectal temperature was monitored through the experiment. A PE50 catheter was placed on the right femoral artery and arterial blood pressure was recorded through a P23XL transducer (Grass Instruments, Quincy, Massachusetts). Peptides and small molecules were injected into the right femoral vein through another catheter. All procedures were performed under a protocol approved by the National Institutes of Health.

Injection of screen selected positive modulators of AM (at 20 nmols/Kg) in hypertensive rats induced a long-lasting decrease in blood pressure (Figures 4A and 4B), when compared to basal levels. These differences were 62 ±21 Hg (p<0.05) for compound 128911 and 55±24 mm Hg (p<0.05) for 145425. Vehicle alone (DMSO in PBS) at the same concentration did not alter blood pressure (Figure 4A). On the other hand, when screen selected negative modulators of AM were injected into normotensive animals, also at 20 nmols/Kg, an elevation in blood pressure was observed (Figure 4C). In the case of compound 16311, the difference from basal levels was 127±47 mm Hg (p<0.01). These data are shown in Figure 4.

### Example 6 - Cord formation assay

Formation of tube-like structures was performed as described in Kubota et al. (1988) J. Cell Biol. 107, 1589-1598; Nam et al. (2003) Phytother. Res. 17, 107-111; and Macpherson et al. (2003) Mol Cancer Ther 2, 845-54. Briefly, a thin layer of Matrigel (Collaborative Biomedical Products, Bedford, Massachusetts) was allowed to polymerize at the bottom of 24-well plates. Bovine retinal microvascular endothelial cells (a gift from Dr Patricia Becerra, NEI, NIH) were resuspended in Human Endothelial-SFM Basal Growth Medium (Invitrogen) and applied to triplicate wells (2 x 10⁵ cells/500 µl medium) in the presence or absence of the test compounds. After an overnight incubation at 37°C, the tubular structures were photographed (3 pictures per well at 10x) and the number of knots per photographic field were counted as a measure of lattice complexity.

As shown in Figure 5, GRP (5 nM) was able to induce cord formation in a culture of endothelial cells grown on Matrigel (Fig. 5A,B) whereas the addition of 0.5 µM of the screen identified compound XV' (77427) greatly reduced the complexity of the tubular lattice (Fig. 5C). The number of knots per photographic field went from 3 ± 1 (control) to 37 ± 5 for the addition of 5nM GRP (p<0.001) and back to 12 ± 4 when GRP and 77427 were added together (compared to control p=0.02, compared to GRP alone p=0.003).

### Example 7 - Directed in vivo angiogenesis assay (DIVAA)

Analysis and quantitation of angiogenesis was done using DIVAA as previously described (Martinez et al. (2002) J Natl Cancer Inst 94, 1226-37). Briefly, 10 mm long surgical-grade silicone tubes with only one end open (angioreactors) were filled with 20 *µ*l of matrigel alone or mixed with GRP and the small molecules at the indicated concentrations. After the matrigel solidified, the angioreactors were implanted into the dorsal flanks of athymic nude mice (NCI colony). After 11 days, the mice were injected iv with 25 mg/ml FITC-dextran (100 *µ*l/mouse, Sigma) 20 min before removing angioreactors. Quantitation of neovascularization in the angioreactors was determined as the amount of fluorescence trapped in the implants and was measured in a HP Spectrophotometer (Perkin Elmer).

As shown in Figure 6, 1 nM GRP exhibits angiogenic potential *in vivo;* and both the small molecule antagonist, compound XV' (77427), and the anti-GRP monoclonal antibody, 2A11, exhibit a dose-dependent inhibition of GRP-induced angiogenesis.

### Example 8 - Proliferation assays

A. Tumor cells (from a lung cancer cell line, H1299) were seeded in 96-well plates at a density of 2.0 x 10⁵ cells per well in serum-free medium containing different concentrations of the test agent, the small molecule inhibitor compound XV' (77427). After 5 days in culture, the number of viable cells per well was estimated by the growth inhibition MTT assay (as reported in Iwai et al. (1999) Lung Cancer 23, 209-22).

Figure 7 shows that compound XV' (77427) exhibits a modest dose-dependent growth inhibitory action on the lung cancer cells.

B. Cells from the cell line A345 were grown in substrate-independent conditions (clonogenic assay) as previously published (Iwai *et al., supra*).

Figure 8 shows that the small molecule inhibitor compound XV' (77427) reduces the number of colonies developed over a period of 3 weeks in soft agar. (The results are represented as percentage growth over the untreated control.)

### Example 9 - Assay of in vivo tumor growth (xenograft experiment)

Thirty female athymic nude mice from the NIH colony in Frederick (MD) were injected subcutaneously with 1.0 x 10⁷ H1299 cells/mouse. Two weeks later, all the mice had developed palpable tumors under the skin and at this time they were randomly divided in three groups. Three times a week, each individual tumor was measured (length, height, thickness) and every mouse received an intratumoral injection, according to their group. Group 1 (control) received 100 *µ*l PBS (negative control); group 2 received 100 *µ*l 0.5 *µ*M compound XV' (77427) in PBS; and group 3 received 100 *µ*l 5 *µ*M compound XV' (77427) in PBS. When the tumor burden became unbearable (larger than 2000 mm³), the mice were sacrificed.

Figure 9 shows that the tumor size was significantly reduced after a single injection of compound XV', and that the tumors all but disappeared after the second injection.

### Example 10 - Statistics

Different treatments were compared with two-tailed Student's t test. P values smaller than 0.05 were considered statistically significant.

### Example 11 - Characterization of the binding between small molecules and AM

### A. Methods

### Surface Plasmon Resonance Assays

Characterization of the binding between AM and the small molecules was performed immobilizing 3 µg of AM on a CM5 sensor chip by N-hydroxysuccinimide activation, followed by covalent amino coupling of the peptide to the surface, using BIAcore 3000 (Piscataway, NJ). The remaining free surface was blocked with 1 mM ethanolamine and the matrix washed with 0.5 M NaCl solution and then re-equilibrated with binding buffer (1:200 DMSO in PBS). Eight different dilutions of each small molecule were prepared in binding buffer with concentrations ranging from 0 to 10 µM and injected from low to high concentration. Each injection was followed by a matrix regeneration step. Mass transfer control experiments were performed by injecting the same concentration of each small molecule at different flow rates (5, 15, and 75 µl/min). The data were then fitted to several models for a kinetic analysis and the binding constants calculated. The best fittings were obtained with a simple 1:1 Langmuir model.

### Receptor binding assays

Binding of ¹²⁵I-AM to Rat2 cells was performed as described in Martinez et al. (1997) Endocrinology 138, 5597-5604. Briefly, 5x10⁴ cells were placed in 24-well plates coated with fibronectin (20 *µ*g/well). When a monolayer was formed, the cell were washed 3 times in transferrin, insulin, and selenium (TIS) medium, followed by incubation with receptor-binding medium (TIS plus 1% BSA and 1 mg/ml bacitracin) with 0.2 nM ¹²⁵I-AM (2200 Ci/mmol, Phoenix) in the presence or absence of competitors (cold AM or small molecules). After 2 h at 4°C, free peptide was removed by washing 3 times in receptor-binding medium. Peptide bound to the cells was solubilized in 0.2 N NaOH and counted in a γ-counter

### B. Results - Characterization of the binding between the small molecules and AM

To determine the mechanism of action of the small molecules, we first performed receptor binding assays and saw no change in the affinity of AM for its receptor in the presence or absence of the small molecule regulators, indicating that these molecules are not receptor modulators. The other possibility is a direct binding to the peptide. This was demonstrated by surface plasmon resonance assays. AM was immobilized into the chip's gold surface and the binding of the small molecules was followed by their effect on the angle of the reflected light. This effect was dose-dependent, allowing for a kinetic analysis of the binding. The calculated KDs varied from 2.93 x 10⁻⁶ for compound 128911 to 6.56 x 10⁻⁹ for compound 16311. One of the molecules identified for its binding to GRP (54671) was used as a control and was shown not to bind to immobilized AM. A summary of the results is presented in Table 2:

**Table 2**

| Characterization of the binding between a few selected small molecules and AM or GRP. ka: kinetic association constant. kd: kinetic dissociation constant. KA: thermodynamic association constant. KD: thermodynamic dissociation constant. | | | | | | | |
|---|---|---|---|---|---|---|---|
| | | | | Binding to AM | | | |
| Analytes | Peptide target | Response to second messengers | Biological activity | ka (1/Ms) | kd (1/s) | KA (1/M) | KD (M) |
| 89435 | AM | Neg. modul. | Vasodilator | 1.02 x 10³ | 8.71 x 10⁻⁴ | 1.18 x 10⁶ | 8.51 x 10⁻⁷ |
| 16311 | AM | Neg. modul. | Vasodilator | 1.55 x 10³ | 1.02 x 10⁻⁵ | 1.52 x 10⁸ | 6.56 x 10⁻⁹ |
| 128911 | AM | Posit. modul. | Vasopressor | 304 | 8.92 x 10⁻⁴ | 3.41 x 10⁵ | 2.93 x 10⁻⁶ |
| 54671 | GRP | Neg. modul. | | No binding | | | |

To examine a potential mass transfer influence, a constant concentration of the small molecule was injected and allowed to react with the immobilized peptide at different flow rates. These experiments clearly demonstrate that binding rates are independent of flow rate and a mass transfer influence could therefore be ruled out.

From the foregoing description, one skilled in the art can easily ascertain the essential characteristics of this invention, and without departing from the scope of the appended claims, can make changes and modifications of the invention to adapt it to various usage and conditions.

Without further elaboration, it is believed that one skilled in the art can, using the preceding description, utilize the present invention to its fullest extent. The preceding preferred specific embodiments are, therefore, to be construed as merely illustrative, and not limitative of the remainder of the disclosure in any way whatsoever.

### References

US patent applications 20020019347, 20020055615 (Cuttitta et al.) and 20020176819; USPs 5,109,115 (Cuttitta), 5,460,801 (Cuttitta), 4,517,188, 5,834,433, 5,047,502, and 5,620,955; EP 806418; Japanese patent JP 10212235; Isumi et al. (1998) Endocrinology 139, 2552-2563; Coppock et al. (1999) Biochem J. 338, 15-22;_Ishizaka et al. (1994) Biochem. Biophys. Res. Comm. 200, 642-646; Heimbrook et al. (1991) J. Med. Chem. 34, 2102-2107; O'Brien et al. (1965) Journal of Medicinal Chemistry 8, 182-7; Pficiderer et al. (1961) Chemische Berichte 94, 2708-21; Gibson et al. (1998) Journal of the American Chemical Society, Perkin Transactions 1: Organic and Bio-Organic Chemistry 18, 3025-30325; Schally et al. (2001) Front. Neuroendocrinol. 22, 248-91; Bajo et al. (2004) Br. Journal of Cancer 90, 245-252; Ganguly et al.(1963) Indian Journal of Chemistry 1(8), 364; Draoui, Dissertation, George Washington University, 1993.

## Claims

1. A complex comprising a compound of one of: 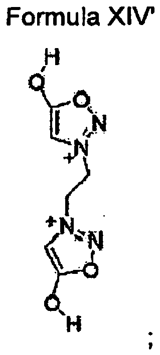 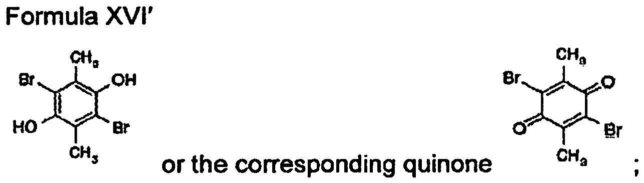 and 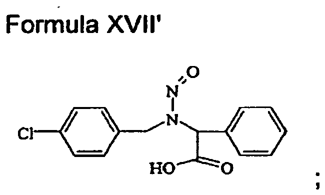 or a pharmaceutically acceptable salt thereof;
in association with a gastrin releasing peptide (GRP).

2. A pharmaceutical composition comprising a compound of formula XVI', as defined in claim 1, or a pharmaceutically acceptable salt thereof, and a pharmaceutically acceptable carrier.

3. A compound of formula XVII' as defined in claim 1, for use in a method of treatment by therapy.

4. A compound of formula XVII' as defined in claim 1 for the use of claim 3, wherein the use is stimulating an activity of a GRP peptide.

5. The compound of formula XVII' as defined in claim 1 for the use of claim 4, wherein stimulating an activity of a GRP peptide provides treatment for a condition that is mediated by under-expression and/or activity of GRP, and/or that would benefit from increased expression and/or activity of GRP, wherein the condition is obesity, diabetes, hypertension, coronary or peripheral artery disease, tissue ischemia, or enhancing of fracture repair or wound healing.

6. A method for detecting a GRP peptide, comprising
(i) contacting a sample suspected of comprising the peptide with one or more detectably labelled compounds of formula XIV', XV', XVI' or XVII';
wherein formula XIV' is: 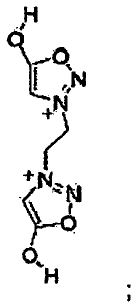 wherein formula XV' is: 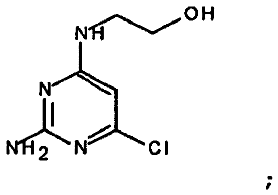 wherein formula XVI' is: 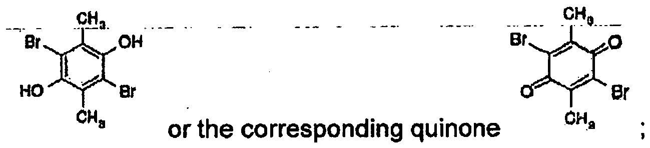 and
wherein formula XVII' is: 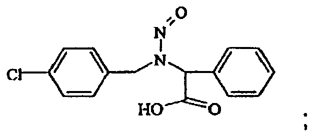 and
(ii) detecting a labelled compound that is associated with the peptide.

7. A compound of formula XIV', XV' or XVI', as defined in claim 6, for use in a method of treatment of a condition that is mediated by over-expression and/or -activity of GRP, wherein the condition is hypotension, an eating disorder, or bronchopulmonary dysplasia in premature babies.

8. The compound of formula XVI' as defined in claim 6, or formula XIV or XV, for use in a method of treatment of a condition mediated by aberrant angiogenesis, wherein the condition is not tumor growth, wherein the condition is selected from the group consisting of: arthritis, psoriasis, brain ischaemia, atherosclerosis, myocardial angiogenesis, post-balloon angioplasty, vascular restenosis, neointima formation following vascular trauma, vascular graft restenosis, coronary collateral formation, deep venous thrombosis, ischemic limb angiogenesis, ocular neovascularization, diabetic ocular neovascularization, neovascular glaucoma, macular degeneration, diabetic and other retinopathy, retrolental fibroplasias, and corneal diseases, fibrosis, and endometriosis;
wherein formula XIV is: 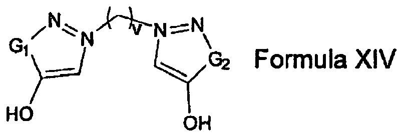
wherein v is an integer from 1-3; and
G₁ and G₂ are the same or different and each is selected from CH₂, NH, S and O; and
wherein formula XV is: 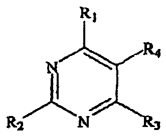 wherein
R₁ is: -R₅-(CH₂)ₙ-CH(R₆)OH, and R₅ is NH, S or O, R₆ is H or CH₃; and n is an integer from 1-4;
R₂ is NH₂, substituted amino or acetamide;
R₃ is H, halogen, CH₃, or CF₃; and
R₄ is H, alkyl, substituted alkyl, alkenyl, alkoxy or halogen.

9. The compound of formula XIV' or XV' as defined in claim 6 for the use of claim 8

10. A compound of formula XV' or XVI' as defined in claim 6, for use in a method of treatment of a neoplastic cellular proliferative disease selected from the group consisting of sarcoma, carcinoma, lymphoma, malignant melanoma, or benign growth caused by rapidly dividing cells.

11. The compound of formula XIV' or XV' as defined in claim 6 for the use of claim 10, wherein the neoplastic cellular proliferative disease is a primary tumor growth, tumor invasion, metastasis, or two or more thereof.

12. The compound of formula XIV' or XV' as defined in claim 6 for the use of claim 10, wherein the neoplastic cellular proliferative disease is a cancer selected from the group consisting of: adrenal, glioma, astrocytoma, neuroblastoma, renal, lung, pancreatic, gastric, gastrointestinal, lung, colon, colorectal, prostate, ovarian, breast, and chondrosarcoma.

13. A non-therapeutic method of preventing or treating a condition mediated by aberrant angiogenesis, wherein the condition is selected from the group consisting of:
benign growths caused by rapidly dividing cells, and wrinkles,
comprising administering to a subject an effective amount of a compound of formula XVI' as defined in claim 6, or formula XIV or XV as defined in claim 8.

## Patentansprüche

1. Komplex, der eine Verbindung mit einer der folgenden Formeln: 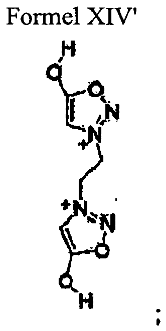 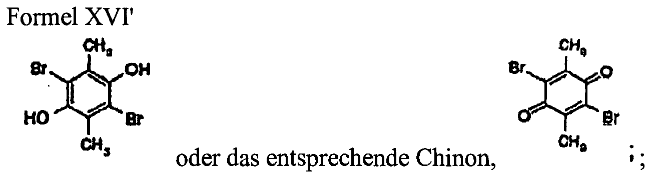 und 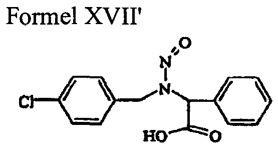 oder ein pharmazeutisch akzeptierbares Salz davon
in Verbindung mit einem Gastrin freisetzenden Peptid (GRP) aufweist.

2. Pharmazeutische Zusammensetzung, die eine Verbindung gemäß Formel XVI', wie in Anspruch 1 definiert, oder ein pharmazeutisch akzeptierbares Salz davon und einen pharmazeutisch akzeptierbaren Träger aufweist.

3. Verbindung gemäß Formel XVII', wie in Anspruch 1 definiert, zur Verwendung bei einem Verfahren zur Behandlung durch Therapie.

4. Verbindung gemäß Formel XVII', wie in Anspruch 1 definiert, zur Verwendung nach Anspruch 3, wobei die Verwendung eine Aktivität eines GRP-Peptids stimuliert.

5. Verbindung gemäß Formel XVII', wie in Anspruch 1 definiert, zur Verwendung nach Anspruch 4; wobei durch Stimulieren einer Aktivität eines GRP-Peptids ein Leiden behandelt wird, das durch Unterexpression und/oder -aktivität von GRP vermittelt wird und/oder das aus erhöhter Expression und/oder Aktivität von GRP Nutzen ziehen würde, wobei das Leiden Fettleibigkeit, Diabetes, Hypertonie, Erkrankung von Koronar- und peripheren Arterien, Gewebeischämie ist, oder die Bruchreparatur oder Wundheilung verbessert wird.

6. Verfahren zum Nachweis eines GRP-Peptids, wobei das Verfahren aufweist:
(i) Inkontaktbringen einer Probe, bei der vermutet wird, dass sie das Peptid aufweist, mit einer oder mehreren nachweisbar markierten Verbindungen gemäß Formel XIV', XV', XVI' oder XVII';
wobei XIV' durch die folgende Formel gegeben ist: 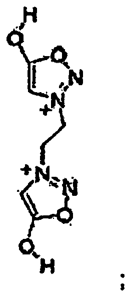 wobei XV' durch die folgende Formel gegeben ist: 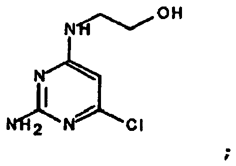 wobei XVI' durch die folgende Formel gegeben ist: 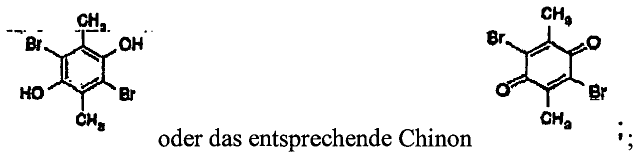 und
wobei XVII' durch die folgende Formel gegeben ist: 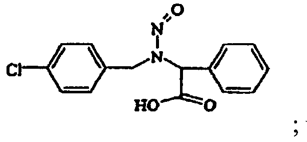 und
(ii) Nachweis einer markierten Verbindung, die mit dem Peptid assoziiert ist.

7. Verbindung gemäß Formel XIV', XV', oder XVI',wie in Anspruch 6 definiert, zur Verwendung bei einem Verfahren zur Behandlung eines Leidens, das durch Überexpression und/oder -aktivität von GRP vermittelt wird, wobei das Leiden Hypotonie, eine Essstörung oder bronchopulmonale Dysplasie bei Frühgeborenen ist.

8. Verbindung gemäß Formel XVI', wie in Anspruch 6 definiert, oder gemäß Formel XIV oder XV, zur Verwendung bei einem Verfahren zur Behandlung eines Leidens, das durch aberrante Angiogenese vermittelt wird, wobei das Leiden kein Tumorwachstum ist, wobei das Leiden aus der Gruppe ausgewählt ist, die aus Arthritis, Psoriasis, Gehirnischämie, Atherosklerose, Myokardangiogenese, post-Ballon-Angioplastik, Gefäßrestenose, Neointimabildung nach Gefäßtrauma, Gefäßtransplantat-Restenose, Bildung von koronaren Kollateralen, tiefe Venenthrombose, Angiogenese in einem ischämischen Glied, okulare Neovaskularisation, diabetische okulare Neovaskularisation, neovaskuläres Glaukom, Makuladegeneration, diabetische und andere Retinopathie, retrolentale Fibroplasien und Hornhauterkrankungen, Fibrose und Endometriose besteht;
wobei XIV durch die folgende Formel gegeben ist: 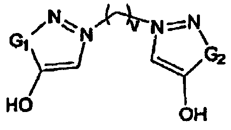
wobei v eine ganze Zahl von 1 bis 3 ist; und
G₁ und G₂ gleich oder verschieden sind und jeweils unter CH₂, NH, S und O ausgewählt sind; und
wobei XV durch die folgende Formel gegeben ist: 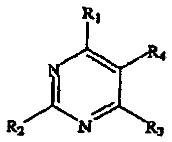
wobei
R₁ für -R₅-(CH₂)ₙ-CH(R₆)OH steht und R₅ für NH; S oder O steht, R₆ für H oder CH₃ steht; und n eine ganze Zahl von 1-4 ist;
R₂ für NH₂, substituiertes Amino oder Acetamid steht;
R₃ für H, Halogen, CH₃ oder CF₃ steht; und
R₄ für H, Alkyl, substituiertes Alkyl, Alkenyl, Alkoxy oder Halogen steht.

9. Verbindung gemäß Formel XIV' oder XV', wie in Anspruch 6 definiert, zur Verwendung gemäß Anspruch 8.

10. Verbindung gemäß Formel XV' oder XVI',wie in Anspruch 6 definiert, zur Verwendung bei einem Verfahren zur Behandlung einer Erkrankung mit neoplastischer Zellproliferation, die aus der Gruppe ausgewählt ist, die aus Sarkom, Karzinom, Lymphom, malignem Melanom oder durch sich schnell teilende Zellen verursachtem benignem Wachstum besteht.

11. Verbindung gemäß Formel XIV' oder XV', wie in Anspruch 6 definiert, zur Verwendung gemäß Anspruch 10, wobei die Erkrankung mit neoplastischer Zellproliferation ein primäres Tumorwachstum, eine Tumorinvasion, Metastasierung oder zwei oder mehr dieser Erkrankungen ist.

12. Verbindung gemäß Formel XIV' oder XV', wie in Anspruch 6 definiert, zur Verwendung gemäß Anspruch 10, wobei die Erkrankung mit neoplastischer Zellproliferation ein Krebs ist, ausgewählt aus der Gruppe, die aus Nebennierenkarzinom, Gliom, Astrozytom, Neuroblastom, Nieren-, Lungen-, Bauchspeicheldrüsen-, Magen-, Magen-Darm-, Dickdarm-, Kolorektal-, Prostata-, Eierstock-, Brustkrebs und Chondrosarkom besteht.

13. Nichttherapeutisches Verfahren zur Verhütung oder Behandlung eines durch aberrante Angiogenese vermittelten Leidens, wobei das Leiden aus der Gruppe ausgewählt ist, die aus durch sich schnell teilende Zellen verursachtem benignem Wachstum und Falten besteht.
wobei das Verfahren aufweist: Verabreichen einer wirksamen Menge einer Verbindung gemäß Formel XVI', wie in Anspruch 6 definiert, oder gemäß Formel XIV oder XV, wie in Anspruch 8 definiert, an einen Patienten.

## Revendications

1. Complexe comprenant un composé parmi un de: 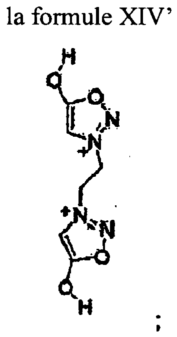 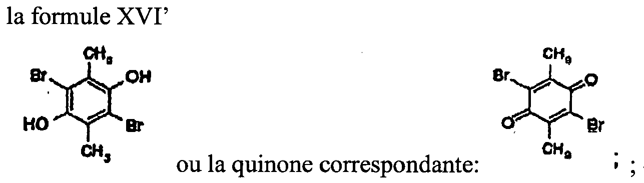 et 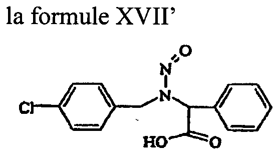 ou un sel pharmaceutiquement acceptable de celui-ci;
en association avec un peptide de libération de gastrine (GRP).

2. Composition pharmaceutique comprenant un composé de la formule XVI', telle que définie dans la revendication 1, ou un sel pharmaceutiquement acceptable de celui-ci, et un support pharmaceutiquement acceptable.

3. Composé de la formule XVII', telle que définie dans la revendication 1, pour une utilisation dans une méthode de traitement par thérapie.

4. Composé de la formule XVII', telle que définie dans la revendication 1, pour l'utilisation selon la revendication 3, dans lequel l'utilisation est la stimulation d'une activité d'un peptide GRP.

5. Composé de la formule XVII', telle que définie dans la revendication 1, pour l'utilisation selon la revendication 4, dans lequel la stimulation d'une activité d'un peptide GRP donne un traitement pour un état qui est induit par une sous-expression et/ou - activité de GRP et/ou qui devrait tirer avantage d'une expression et/ou d'une activité de GRP accrue, dans lequel l'état est une obésité, un diabète, une hypertension, une maladie d'artère coronaire ou périphérique, une ischémie tissulaire ou une augmentation de réparation de fracture ou de guérison de blessure.

6. Méthode pour la détection d'un peptide GRP, comprenant:
(i) la mise en contact d'un échantillon suspecté de comprendre le peptide avec un ou plusieurs composés marqués de façon détectable de la formule XIV', XV', XVI' ou XVII';
dans laquelle la formule XIV' est: 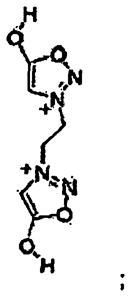 dans laquelle la formule XV' est: 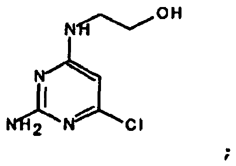 dans laquelle la formule XVI' est: 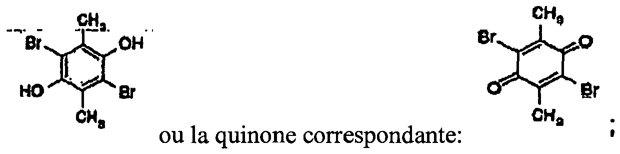 et
dans laquelle la formule XVII' est: 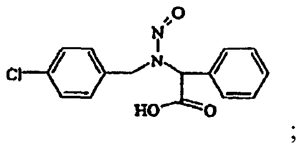 et
(ii) la détection d'un composé marqué qui est associé au peptide.

7. Composé de la formule XIV', XV' ou XVI', telle que définie dans la revendication 6, pour une utilisation dans une méthode de traitement d'un état qui est induit par une sur-expression et/ou -activité de GRP, dans lequel l'état est une hypotension, un trouble de l'alimentation ou une dysplasie broncho-pulmonaire chez des bébés prématurés.

8. Composé de la formule XVI', telle que définie dans la revendication 6, ou de la formule XIV ou XV, pour une utilisation dans une méthode de traitement d'un état induit par une angiogenèse aberrante, dans lequel l'état n'est pas une croissance de tumeur, dans lequel l'état est choisi dans le groupe constitué de: arthrite, psoriasis, ischémie cérébrale, athérosclérose, angiogenèse du myocarde, angioplastie post-ballonnet, resténose vasculaire, formation de néo-intima à la suite d'un traumatisme vasculaire, resténose de greffe vasculaire, formation collatérale coronaire, thrombose veineuse profonde, angiogenèse de membre ischémique, néo-vascularisation oculaire, néo-vascularisation oculaire diabétique, glaucome néo-vasculaire, dégénérescence maculaire, rétinopathie diabétique et autre, fibroplasies rétrolentales et maladies de la cornée, fibrose et endométriose;
dans lequel la formule XIV est: 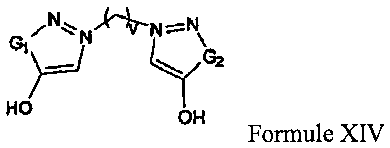
dans laquelle v est un nombre entier de 1 à 3; et
G₁ et G₂ sont identiques ou différents et chacun est choisi parmi CH₂, NH, S et O; et
dans lequel la formule XV est: 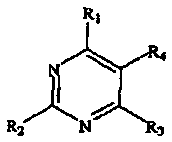 dans laquelle:
R₁ est: -R₅-(CH₂)ₙ-CH(R₆)OH, et R₅ est NH, S ou O, R₆ est H ou CH₃; et n est un nombre entier de 1 à 4;
R₂ est NH₂, un amino substitué ou un acétamide;
R₃ est H, un halogène, CH₃ ou CF₃; et
R₄ est H, un alkyle, un alkyle substitué, un alcényle, un alcoxy ou un halogène.

9. Composé de la formule XIV' ou XV', telle que définie dans la revendication 6, pour l'utilisation selon la revendication 8.

10. Composé de la formule XV' ou XVI', telle que définie dans la revendication 6, pour une utilisation dans une méthode de traitement d'une maladie proliférative cellulaire néoplasique choisie dans le groupe constitué d'un sarcome, d'un carcinome, d'un lymphome, d'un mélanome malin ou d'une croissance bénigne entraînée par des cellules se divisant rapidement.

11. Composé de la formule XIV' ou XV', telle que définie dans la revendication 6, pour l'utilisation selon la revendication 10, dans lequel la maladie proliférative cellulaire néoplasique est une croissance de tumeur primaire, une invasion de tumeur, une métastase ou deux ou plus de celles-ci.

12. Composé de la formule XIV' ou XV', telle que définie dans la revendication 6, pour l'utilisation selon la revendication 10, dans lequel la maladie proliférative cellulaire néoplasique est un cancer choisi dans le groupe constitué de: surrénal, gliome, astrocytome, neuroblastome, rénal, du poumon, pancréatique, gastrique, gastro-intestinal, du poumon, du côlon, colorectal, de la prostate, ovarien, du sein et chondrosarcome.

13. Méthode non thérapeutique pour la prévention ou le traitement d'un état induit par une angiogenèse aberrante, dans laquelle l'état est choisi dans le groupe constitué de: croissances bénignes entraînées par des cellules se divisant rapidement et rides,
comprenant l'administration à un sujet d'une quantité efficace d'un composé de la formule XVI' telle que définie dans la revendication 6 ou de la formule XIV ou XV telle que définie dans la revendication 8.
